# EUROPEAN PATENT APPLICATION

(11) **EP 4 847 001 A2**
(43) Date of publication of application: **02.12.2026**
(21) Application number: 25713986.5
(22) Date of filing: 27.01.2025
(51) Int. Cl.: A61K 8/02, A61K 8/9789, A61Q 19/00

(54) **CORK POWDER, AQUEOUS EXTRACT, METHODS AND USES THEREOF**

(30) Priority: 26.01.2024 PT 2024119223; 26.01.2024 PT 2024119224
(71) Applicant: Dimas e Silva Lda, 4535-167 Mozelos (PT); Universidade Do Porto, 4099-002 Porto (PT)
(72) Inventor: MARTINS DE ALMEIDA, Isabel Filipa, 4050-313 Porto (PT); DE MATOS CIDADE, Honorina Maria, 4050-313 Porto (PT); TIRITAN, Maria Elizabeth, 4050-313 Porto (PT); FERREIRA PINTO, Cláudia Sofia, 4050-313 Porto (PT); MENDONÇA DA SILVA CRAVO, Sara Manuela, 4050-313 Porto (PT); SILVA REGO, Liliana Sofia, 4050-313 Porto (PT); MAGALHÃES AFONSO, Carlos Manuel, 4050-313 Porto (PT); RAMOS TORRES, Ana Cristina, 4050-313 Porto (PT); NETO FERREIRA DE SOUSA, Helena Maria, 4050-313 Porto (PT); DA SILVA MOTA, Sandra Filipa, 4050-313 Porto (PT); CABRAL DE SOUSA E SILVA, José Paulo, 4050-313 Porto (PT); CORREIA DAS NEVES SOUSA LOBO, José Manuel, 4050-313 Porto (PT)
(74) Representative: Patentree
(86) International application number: PCT/IB2025/050896
(87) International publication number: WO 2025/158419

(57) **Abstract**

The present disclosure relates to a cork powder and an aqueous extract thereof and to a method for obtaining said powder and said extract. Furthermore, the present application relates to compositions comprising said powder and/or said extract and to the use thereof in cosmetics or medicine.

## Description

### TECHNICAL FIELD

The present disclosure relates to a cork powder and an aqueous extract thereof and a method for obtaining said powder and said extract. Furthermore, the present application relates to compositions comprising said powder and/or said extract and to the use thereof in cosmetics or medicine.

### BACKGROUND

The cork industry's main raw material is cork, which is used for various purposes, including the production of cork stoppers. The processing of cork results in the production of a considerable amount of waste, such as fragments, pieces and low-quality cork. Since raw material waste represents a high percentage of the product obtained, it is crucial to use it in other industries or for the production of different materials. Therefore, this waste is often used in the manufacture of agglomerates for various purposes, such as disks, covering boards, fixing and decoration panels, insoles, among others. However, approximately 25% of the initial cork is transformed into very small granulometries from the various stages of its processing. This fraction is known as cork powder.

Cork powder comes from various stages of cork processing:
- A: Crushing powder - powder originating in the initial phase of granulation operations, consisting of several phases (pre-crushing and drying) prior to cleaning and separation operations;
- B: Cleaning powder - powder obtained in the next phase involving separation - cleaning the back and belly of cork and other impurities;
- C: Powder from granulometric separations - powder produced in the final phase of granulation, in the separation of granulates by different granulometry;
- D: Powder from the finishing of agglomerate panels - Obtained from laminating, sanding and cutting of agglomerate panels;
- E: Powder from the finishing of cork stoppers and discs - Originates from sanding, shaping, chamfering and thickness adjustment operations;
- F: Powder from the finishing of natural cork stoppers - Originates from sanding, shaping and chamfering operations (Gil, Luis & santos, J. & Florêncio, M.I. (1986). *Identifica* 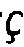 *ão e caracteriza* 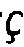 *ão de vários tipos de pó obtidos no processamento industrial do corti* 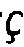 *a. Boletim IPF-Corti* 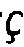 *a.* 255-261).

Cosmetic companies are currently implementing new procedures based on balancing the economy, environment and society in order to offset their environmental footprint while addressing customer concerns about sustainability and the exploitation of natural resources. As technological innovation advances, the potential and appeal of using upcycled elements in cosmetics is expanding. Cork by-products are a unique sustainable source due to four factors: regeneration of cork bark (every 9 years), longevity of *Q. suber* (up to 250 years), substantial environmental effect of Q. *suber* ecosystems (cork oak forests), and production of a large volume of cork powder (per product).

The use of cork powder or an extract obtained from cork powder and its application in value added products, particularly in the cosmetic industry, would allow this by-product to be valorized, contributing to the sustainable management of natural resources. However, its application in a cosmetic product presents a number of challenges:
- Odor and color: Cork powder or powder extract have their own odor and color characteristics, which have an impact on the sensory characteristics and sensory acceptance by consumers;
- Compatibility: Cork powder or powder extract may not be compatible with other ingredients present in cosmetic formulations, leading to a formulation with poor stability.

In addition, the process of obtaining cork powder or cork powder extract can lead to the degradation of bioactive compounds, losing their biological action and the benefits of incorporating them into a cosmetic composition.

These facts are described in order to illustrate the technical problem solved by the embodiments of the present document.

### GENERAL DESCRIPTION

The present disclosure relates to a cork powder and an aqueous extract thereof, and to a method for obtaining said powder and said extract. Furthermore, the present application also relates to compositions comprising said powder and/or said extract and as the use thereof in cosmetics or medicine.

The cork powder of the present disclosure is a light brown, homogeneous fine powder, that surprisingly has been shown to have the ability to synergistically improve the *sun* protection factor (SPF) of sunscreens comprising titanium dioxide (increase of at least 2 times), while transmitting a natural color and being compatible with the skin. It also has excellent oil absorption properties (five times greater than kaolin) as well as low moisture content and microbiological quality, and still a suitable volatile compound profile.

The cork powder of the present disclosure can be included in solid, liquid and semi-solid formulations, such as purifying peel-off masks, tinted sunscreens, dry shampoos, compacts and mattifying make-up products, among others.

The method for obtaining the cork powder of the present disclosure, in particular the method for washing the powder and reducing the particle size and the method for reducing the microbial load, has been optimized in such a way as to make it possible to obtain a homogeneous and stable powder with microbiological characteristics suitable for cosmetic or medical use. The method also proved to be reproducible and easily scalable.

The present disclosure addresses the need of the cosmetics industry and other industries for sustainable ingredients by providing an ingredient of natural origin, obtained from an agro industrial by-product, produced in relevant quantities with consistent quality and obtained with a zero-waste process.

The multifunctionality of the cork powder of the present disclosure (fragrance, color, oil absorption and SPF boost) is also appealing in terms of reducing the use of additional ingredients in the same formulation, thus reducing the carbon footprint of the products in which it is used.

Another aspect of the present disclosure relates to a cork powder extract. Surprisingly, the inventors of the present application have obtained a cork powder extract with unique characteristics, which comprises the bioactive compounds of cork through the development of an innovative extraction method, which allows the cork powder extract obtained to have unique characteristics and to comprise the bioactive compounds of cork and preserve their activity. In addition, the cork powder extract has the right sensory characteristics, namely odor and color, as well as stability. The cork powder extract obtained has antioxidant, anti-senescence and anti-inflammatory properties.

The method of obtaining the extract developed is an ecological and sustainable method that is easy to scale up to an industrial scale, since it can be carried out at room temperature, allows the extraction solvent to be water and also allows the extract to be obtained with just one extraction cycle.

HPLC analysis of the cork extract showed that the extract has a high phenolic content. The safety profile of the extract of the present disclosure has also been confirmed, on cells of the human epidermis and reconstructed human epidermis (Episkin). The source material also has a reproducible profile presenting consistent quality of the cosmetic/pharmaceutical ingredient. The extract of the present disclosure showed antioxidant, anti-inflammatory and anti-aging effects, demonstrating application in the prevention and treatment of inflammation. In addition, the extract was found to have adequate microbiological quality and stability. The overall results demonstrate the unique properties of this extract and its application as an active ingredient for incorporation into a cosmetic product or use as a medicine.

The extract of the present disclosure is unique, since this aqueous extract of cork powder showed antioxidant potential by increasing the expression of the antioxidant genes NQO1 (NAD(P)H Quinone Dehydrogenase 1) and HMOX1 (Heme Oxygenase 1), and increased the levels of HMOX protein (Heme Oxygenase) *per se* and under an inflammatory environment (macrophages exposed to LPS (lipopolysaccharide). This extract, in an inflammatory environment (i.e. cells exposed to LPS) induced a significant reduction in the expression of the Nos2 gene (Nitric Oxide Synthase 2), reinforcing the anti-inflammatory potential of this aqueous extract of cork powder.

An aspect of the present disclosure relates to a cork powder for use in cosmetics or medicine (human or veterinary) comprising cork particles with a particle size of less than 100 µm, and wherein the color has values of L* between 45-55; of a* between 6-10 and of b* between 12-16, measured by the CIELAB system.

In a preferred embodiment, the cork powder comprises cork particles with a particle size of less than 63 µm; preferably ranging between 16-63 µm; more preferably 20-40 µm.

In a preferred embodiment, the cork powder is a powder that has been subjected to 180 °C for at least 30 minutes; preferably 30 minutes (sterilization process).

In a preferred embodiment, the oil absorption capacity of cork powder is at least 4 times higher than that of kaolin (measured in g/100g by ISO 787/5-1980).

Another aspect of the present disclosure relates to a method of obtaining cork powder comprising the following steps:
supplying cork powder comprising cork particles with a particle size of less than 100 µm; preferably less than 63 µm; more preferably ranging between 16-63 µm; even more preferably ranging between 20-40 µm;
adding water and stirring for at least 15 minutes (preferably 15-20 minutes at 1250 rpm) in order to obtain a dispersion of cork powder; preferably wherein the mass ratio of cork powder (g): aqueous solvent (mL) varies between 1:10 and 1:20; more preferably 1:16;
filtering the cork powder dispersion through a porous glass filter in order to obtain clean cork powder; preferably the porous glass filter is type G4;
drying the clean cork powder at least at 50 °C for at least 1 hour to obtain dry cork powder;
heating the dry cork powder at 180 °C for at least 30 minutes in order to obtain the cork powder as described in this disclosure.

In a preferred embodiment, the cork powder of the method described herein comprises cork particles with a particle size ranging between 16-63 µm; preferably 20-40 µm.

The cork powder described in the present disclosure can be obtained at various stages of cork processing. In a preferred embodiment, the cork powder of the present disclosure is selected from crushing powder; cleaning powder; powder from granulometric separations; powder from the finishing of agglomerate panels; powder from the finishing of cork stoppers and discs; powder from the finishing of natural cork stoppers; or a mixture thereof. Preferably, the powder of the present disclosure is obtained in the phase of granulometric separations (powder from granulometric separations).

Another aspect of the present disclosure relates to an aqueous extract of cork powder for cosmetic or medical use comprising:
castalagin, at a concentration of more than 10 µg/mg (dry extract);
gallic acid, at a concentration of more than 2 µg/mg (dry extract);
ellagic acid, at a concentration of more than 4 µg/mg (dry extract).

In a preferred embodiment, the extract is obtained from the cork powder described in the present disclosure, preferably from a cork powder comprising cork particles with a particle size of less than 100 µm, and wherein the color has L* values between 45-55; a* values between 6-10 and b* values between 12-16, as measured by the CIELAB system.

In a preferred embodiment, the aqueous extract of cork powder is an aqueous extract of freeze-dried cork powder.

In a preferred embodiment, the aqueous extract of cork powder comprises:
castalagin, at a concentration ranging between 10-60 µg/mg (dry extract), preferably between 10-50 µg/mg (dry extract);
gallic acid, at a concentration ranging between 2-20 µg/mg (dry extract), preferably between 2-15 µg/mg (dry extract);
ellagic acid, at a concentration ranging between 4-30 µg/mg (dry extract), preferably between 4-20 µg/mg (dry extract).

In an embodiment, the color of the extract has an L* between 40-60; an a* between 2 and 8 and a b* between 10-20, measured by the CIELAB system.

Another aspect of the present disclosure relates to a cosmetic or pharmaceutical composition comprising the cork powder as described in the present disclosure or the aqueous extract of cork powder as described in the present disclosure.

In a preferred embodiment, the composition comprises 0.1-10 wt % cork powder. In a preferred embodiment, the composition comprises 1-7 wt % cork powder; more preferably 4-6 wt % cork powder; even more preferably 5 wt %.

In a preferred embodiment, the composition comprises 0.1-4 wt% cork powder; preferably 1-3 wt%, cork powder; more preferably 2 wt%.

In an embodiment, the composition further comprises titanium dioxide; preferably 1-15 wt% titanium dioxide; more preferably 8-12 wt%.

Surprisingly, a synergistic effect was found between the cork powder of the present disclosure and the titanium dioxide, wherein the mixture of these two components allows to obtain a higher sun protection factor (at least twice as high).

In an embodiment, the composition comprises 1-7 wt% cork powder; preferably 5 wt%; and 1-15 wt% titanium dioxide; preferably 10 wt%.

In a preferred embodiment, the composition further comprises:
a mixture of glyceryl monostearate, cetostearyl alcohol, cetyl palmitate and coco-glycerides; preferably 2-8 wt %;
ethoxylated cetostearyl alcohol (ceteareth-12); preferably 0.1-3 wt %;
medium-chain triglycerides; preferably 4-12 wt%;
cetearyl isononanoate; preferably 4-12 wt%.

In a preferred embodiment, the sun protection factor of the composition is at least 40, preferably at least 50; more preferably at least 60, even more preferably between 40-80. (measured by a method described in the scientific literature: Pissavini M, Tricaud C, Wiener G, Lauer A, Contier M, Kolbe L, Trullás Cabanas C, Boyer F, Nollent V, Meredith E, Dietrich E, Matts PJ. Validation of an in vitro sun protection factor (SPF) method in blinded ring-testing. International Journal of Cosmetic Science, 2018 Apr 20. doi: 10.1111/ics.12459).)

In a preferred embodiment, the composition further comprises:
2-8 wt% of a mixture of polyglyceryl-6 esters of olive oil, sodium stearoyl lactate, cetostearyl alcohol;
4-12 wt% of dicaprylyl ether;
1-4 wt% of squalene;
1-3 wt % of caprylic/capric acid triglycerides;
1-3 wt% of stearic acid;
1-3 wt% of sweet almond oil;
0.1-0.5 wt% of xanthan gum
1-3 wt% of propanediol
2-8 wt% of Glycerin
0.1-2 wt% of a mixture of phenoxyethanol, benzyl alcohol, ethylhexylglycerin
and tocopherol.

In a preferred embodiment, the color of this cosmetic composition has an L* between 40-54; an a* between 4-8 and a b* between 10-17, measured by the CIELAB system.

The cork powder and extract of the present disclosure are compatible with the skin and do not exhibit irritant potential (as assessed by the OECD 439 test).

Another aspect of the present disclosure relates to a cosmetic or pharmaceutical composition comprising the aqueous extract of cork powder of the present disclosure. In a preferred embodiment, the composition comprises 1-2 wt% aqueous extract of cork powder. In an even more preferred embodiment, the composition comprises 0.1-2 wt % aqueous extract of cork powder; preferably 0.1-1 wt%; more preferably 0.5 -1 wt %.

In an embodiment, the composition comprising the aqueous extract of cork powder further comprises:
4-6 wt% of a mixture of polyglyceryl-6 esters of olive oil, sodium stearoyl lactate, cetostearyl alcohol;
10-20 wt% of triglycerides of capric acid and/or caprylic acid;
1-3 wt% of cetostearyl alcohol;
0.1-0.2 wt% of xanthan gum;
1-3 wt% of propylene glycol;
0.1-2 wt% of a mixture of phenoxyethanol, benzyl alcohol, ethylhexylglycerin
and tocopherol.

In a preferred embodiment, the color of the composition comprising the aqueous extract of cork powder has an L* between 48-55; an a* between 2 and 7 and a b* between 15-20, measured by the CIELAB system.

The composition of the present disclosure may be presented in different pharmaceutical forms. In a preferred embodiment, the composition of the present disclosure is selected from a list comprising: solution, serum, cream, lotion, gel, hydrogel, oleogel, oil, soap, shampoo, dry shampoo, spray, ointment, paste, mousse or body wash. In an even more preferred embodiment, the composition is a cream.

Another aspect of the present disclosure relates to the use of cork powder or the composition comprising the cork powder as an oil-absorbing agent.

Another aspect of the present disclosure relates to the use of cork powder or the composition comprising the cork powder as a pigment; preferably as a cosmetic pigment.

Another aspect of the present disclosure relates to the use of cork powder or the composition comprising the cork powder as a sun protection factor enhancer, preferably wherein the enhancing factor is at least 2 times; more preferably the enhancing factor ranges between 2-3 times.

Another aspect of the present disclosure relates to the use of cork powder or the composition comprising the cork powder as an ultraviolet radiation blocking agent.

Another aspect of the present disclosure relates to the use of cork powder or the composition comprising the cork powder in medicine or veterinary. In a preferred embodiment, the cork powder or the composition is used in the prevention and treatment of skin diseases; preferably skin diseases caused by exposure to the sun. In an embodiment, the cork powder or the composition is used in the prevention and/or treatment of skin cancer.

Another aspect of the present disclosure relates to the use of the extract or the composition comprising the extract as a skin ageing preventive or retarding agent.

Another aspect of the present disclosure relates to the use of the extract or the composition comprising the extract as an anti-senescent agent (anti-aging).

Another aspect of the present disclosure relates to the use of the extract or the composition comprising the extract as an antioxidant agent.

Another aspect of the present disclosure relates to the use of the extract or the composition comprising the extract for use in medicine. In a preferred embodiment, it relates to the use of the extract or the composition comprising the extract either for use in the prevention or treatment of inflammation, and/or for use as an anti-inflammatory; preferably topical anti-inflammatory.

Another aspect of the present disclosure relates to a method for obtaining the extract described herein comprising the following steps:
providing cork powder comprising cork particles with a particle size of less than 0.5 mm;
adding an aqueous solvent to the cork powder in a mass ratio of cork powder(g): aqueous solvent(mL) that ranges between 1:20 and 1:80, and stirring for at least 30 minutes to obtain a mixture comprising the aqueous extract of cork powder;
filtering the mixture comprising the aqueous extract of cork powder to obtain an aqueous extract of cork powder.

In a preferred embodiment, the method comprises an additional step of drying and freeze-drying the aqueous extract of cork powder in order to obtain a freeze-dried aqueous extract of cork powder.

In a preferred embodiment, the granulometry of the cork particles to obtain the extract is less than 250 µm; preferably less than 200 µm; more preferably between 63-200 µm; more preferably 100-200 µm.

In a preferred embodiment, the granulometry of the cork particles to obtain the extract is less than 100 µm.

In a preferred embodiment, the mass ratio of cork powder: aqueous solvent ranges between 1:30 and 1:50. In an even more preferred embodiment, the mass ratio of cork powder: aqueous solvent is 1:40.

In a preferred embodiment, the step of stirring the aqueous solvent and the cork powder ranges between 500-1000 rpm; preferably 700 rpm.

In a preferred embodiment, the aqueous solvent is water.

In a preferred embodiment, the method is carried out at room temperature (20-25 °C).

In a preferred embodiment, the stirring time of the aqueous solvent and the cork powder ranges from 30 - 120 minutes; preferably 60 minutes.

In a preferred embodiment, the filtration step is carried out with a glass fiber filter membrane; preferably with a diameter of 47 mm and a pore size of 1.2 µm.

In a preferred embodiment, the extraction step (adding an aqueous solvent to the cork powder in a mass ratio of cork powder: aqueous solvent ranging between 1:20 and 1:80, and stirring for at least 30 minutes to obtain a mixture comprising the aqueous extract of cork powder) is carried out in a single extraction cycle.

The cork powder used to obtain the cork powder extract can come from different stages of cork processing. In a preferred embodiment, the cork powder is selected from: crushing powder, cleaning powder, powder from granulometric separations, powder from the finishing of agglomerate panels, powder from the finishing of cork stoppers and discs; powder from the finishing of natural cork stoppers, or a mixture thereof. In an even more preferred embodiment, the cork powder is powder from granulometric separations.

### BRIEF DESCRIPTION OF THE DRAWINGS

For ease of understanding, figures are herein attached, which represent preferred embodiments that are not intended to limit the object of this description.
**Figure 1****:** Production process and origin of the by-products under study.
**Figure 2** - Powder from granulometric separations, preferably obtained in the process of dedusting and transporting of granules, with a granulometry between 0-2 mm. Also known as PT powder (transport powder) or P1 powder)
**Figure 3****.** NIR (a) and MIR (b) spectra of P0 powder and P1 powder.
**Figure 4** **-** Score plots obtained from PCA *(Principal Component Analysis*) using NIR spectra of different batches of P0 and P1 powders pre-processed with the Savitzky-Golay filter (filter width of 15 points, second degree polynomial, first derivative) followed by standard normal variant (SNV) and mean center.
**Figure 5****:** Incorporation of the different by-products into a semi-solid formulation.
**Figure 6****.** Determination of the SPF-enhancing capacity of C1E powder.
**Figure 7****.** Analysis of the chemical profile of C1 powder and sterilized C1 powder (C1E). a) NIR; b) MIR.
**Figure 8****.** Mixture of different concentrations of C1 with pigments used in cosmetics decorative. a) 1 wt% C1E powder + 2 wt % red iron oxide + 4.5 wt %, titanium dioxide; b) 2 wt %, C1E powder + 5.5 wt% titanium dioxide; c) 5 wt% C1E powder + 2.5 wt % titanium dioxide.
**Figure** 9. Examples of several different formulations tested, containing different concentrations of cork powder.
**Figure 10****.** Viscosity curve of the base formulation of a colored cream.
**Figure 11****.** Texturogram of the spreadability test of the C1E powder base formulation stored at 25 °C (black) and 40 °C (blue) for 15 days (n=1).
**Figure 12****.** Skin irritation test results.
**Figure 13****.** Appearance of P1 powder.
**Figure 14****.** Color analysis of C1E powder over 90 days at two different temperatures: 25 and 40 °C.
**Figure 15****.** Evolution of C1E powder pH over 90 days of storage at two different temperatures: 25 and 40 °C. n=3.
**Figure 16****.** Analysis of the chemical profile of C1E powder over 90 days and stored at different temperatures: 25 and 40°C - using Near Infrared Spectroscopy (NIR).
**Figure 17****.** Analysis of the chemical profile of C1E powder over 90 days and stored at different temperatures: 25 and 40 °C - using Mid-Infrared Spectroscopy (MIR).
**Figure 18****.** Analysis of the moisture content of C1E powder over 90 days and stored at different temperatures: 25 and 40 °C (n=3).
**Figure 19****.** Stability analysis of the C1E powder formulation, for 90 days, at 25 and 40 °C - Appearance and odor.
**Figure 20****.** Color parameters after storing the BBc05 formulation, for 90 days, at 25 and 40 °C - Color (n=3).
**Figure 21****.** Analysis of the pH of the C1E powder formulation, for 90 days, at 25 and 40 °C (n=3).
**Figure 22****.** Analysis of the stability of the BBc05 formulation, for 90 days, at 25 and 40 °C with regard to flow.
**Figure 23****.** Variation in extract mass with the number of extractions.
**Figure 24****.** Masses obtained for the different extracts (A); Evaluation of the effectiveness of the extraction according to the absorbance of the extract at 280 nm (B) and 350 nm (C).
**Figure 25****.** DPPH radical scavenging activity. Results expressed as mean ± standard error, n= 3; ascorbic acid (positive control): IC₅₀ = 7.9 ± 0.4 µg/mL.
**Figure 26****.** Effect of cork extracts on cell metabolism. Macrophages (A), fibroblasts (B) and keratinocytes (C) were cultured and exposed to different concentrations of cork extracts for 24 hours. The Alamar blue assay was also carried out to assess cell metabolism. The data correspond to the mean ± SEM of at least three independent experiments and are represented as % of control cells (Ctr, black bars).
**Figure 27****.** Effect of cork extracts on NO production in macrophages stimulated with the pro inflammatory mediator LPS. The cells were plated and exposed to 62.5 and 125 µg/mL of cork extracts for 24 h, in the presence or absence of LPS (100 ng/mL). The Griess assay (graphs letter A) and the Alamar blue assay (graphs letter B) were carried out to assess nitrite levels in the supernatant and in cell metabolism (after exposure to LPS), respectively. The data correspond to the mean ± SEM of at least three independent experiments and are represented in nitrite concentration (µM).
**Figure 28****.** Effect of cork extracts on gene expression related to the inflammatory response. Macrophages were cultured and exposed to 125 µg/mL of cork extracts, and to the commercial compound Oleoactif, in the presence or absence of LPS (100 ng/mL) for 6 h. The expression of the pro-inflammatory genes Nos2, Il1β, Tnfα, II6 and the anti-inflammatory gene II10 was determined by real-time RT-PCR. Data correspond to the mean ± SEM of at least three independent experiments and are expressed relative to control cells (Log2=0).
**Figure 29****.** Effect of cork extracts on the expression of genes encoding antioxidant proteins. Keratinocytes were cultured and exposed to 125 µg/mL of cork extracts and to the commercial compound Oleoactif for 6 h. The expression of the antioxidant genes HMOX1, NQ01 and TRX was determined by real-time RT-PCR. Data correspond to the mean ± SEM of at least three independent experiments and are expressed relative to control cells (Log2=0).
**Figure 30****.** Effect of cork extracts on levels of the pro-inflammatory enzyme iNOS. Macrophages were cultured and exposed to 125 µg/mL of P1 cork extracts, and to the commercial compound Oleoactif, in the presence or absence of LPS (100 ng/mL) for 24 h. The levels of iNOS protein were determined by Western-blotting. Representative images of the blots are shown. Data correspond to the mean ± SEM of at least three independent experiments and are expressed as % of LPS.

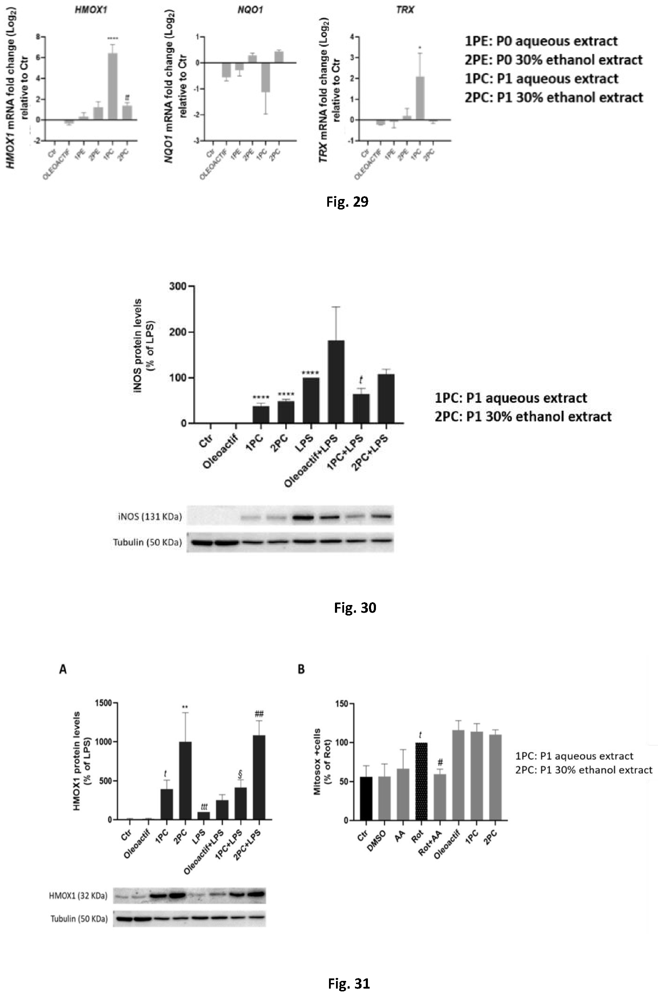

**Figure 31****.** Antioxidant effect of cork extracts on immune cells. Macrophages were cultured and exposed to 125 µg/mL of cork extracts, and to the commercial compound Oleoactif, in the presence or absence of LPS (100 ng/mL) for 24 h (A) or rotenone (20 µM) for 6 h (B). (A) Levels of the antioxidant enzyme HMOX1 were determined by Western-blotting. Representative images of the blots are shown. (B) Superoxide anion (O₂⁻) levels were determined by the number of cells positive for Mitosox red, assessed by flow cytometry. Data correspond to the mean ± SEM of at least three independent experiments and are expressed as % of LPS (A) or Rot (B).

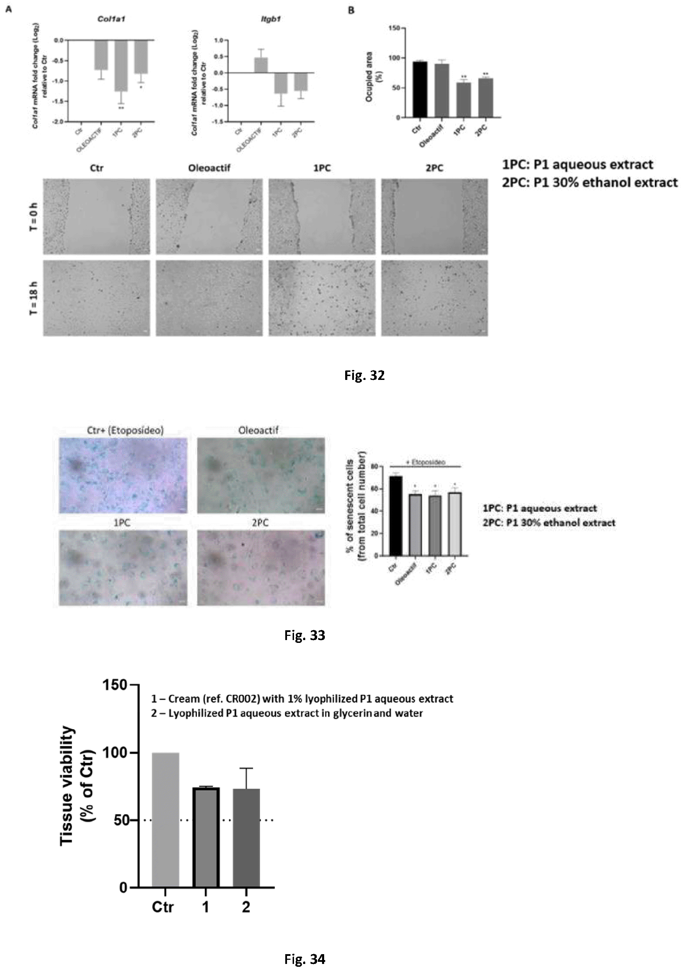

**Figure 32****.** Effect of cork extracts on skin regeneration. Fibroblasts were cultivated and exposed to 125 µg/mL of cork extracts, and to the commercial compound Oleoactif, for 6 h (A) or 18 h (B). (A) The expression of genes encoding for collagen type I (Col1a1) and Integrin beta-1 (Itgb1) proteins was determined by real-time RT-PCR. (B) The scratch wound assay was carried out to assess the effect of cork extracts on cell migratory capacity and skin regeneration. The area occupied was calculated and the results represented in the graph. Representative microscopic images are illustrated in the panel (scale bar = 50 µm). Data correspond to the mean ± SEM of at least three independent experiments and are expressed relative to the control (Ctr).
**Figure 33****.** Anti-senescence effect of cork extracts. Fibroblasts were cultured and exposed to Etoposide (Ctr+; 12.5 µM), a senescence inducer, for 24 h, and the cells were subsequently subjected to a recovery period in the presence of 125 µg/mL of P1 cork extracts, and the commercial compound Oleoactif, for 72 h. Cell senescence was assessed using a staining kit with commercially available beta-galactosidase. The number of senescent cells (stained blue as a result of beta-galactosidase activity) was visualized by microscopy. Representative microscopic images are shown (scale bar = 20 µm). Data correspond to the mean ± SEM of at least three independent experiments and are expressed relative to the control (Ctr).
**Figure 34****.** Effect of the aqueous P1 extract on the EpiSkin skin irritation test.
**Figure 35****.** NIR spectrum of the aqueous P1 extract.
**Figure 36****.** MIR spectrum of aqueous P1 extract.
**Figure. 37** Recording of the appearance of the aqueous P1 extract over time (time (T) in days).
**Figure 38****.** Variation of the chromaticity coordinates of the aqueous P1 extract over time at temperatures of 25 °C and 40 °C (time (T) in days).
**Figure 39****.** Variation in the pH of the aqueous P1 extract over time at temperatures of 25° C and 40° C (time (T) in days).
**Figure 40****.** Overlay of the NIR and MIR spectra of the aqueous P1 extract obtained during the stability test at 25° C and 40° C (time (T) in days).
**Figure 41****.** Evolution of the DPPH scavenging activity (IC₅₀) of the aqueous extract over the storage time (n=3) (time (T) in days).
**Figure 42****.** Evolution of the DPPH scavenging activity (IC₅₀) of the hydroglycerin solution over the storage time at the temperature of 25 °C, n=3 (time (T) in days).
**Figure 43****.** Evolution of the pH of the aqueous extract in hydroglycerin solution (time (T) in days).
**Figure 44****.** Evolution of DPPH scavenging activity (IC₅₀) and pH of the aqueous extract in buffer solutions pH 5 and 7 stored at 25 °C. n=3 (time (T) in days).
**Figure 45****.** Texturograms of CR002 cream stored for 15 days at 25 (A) and 40 °C (B).
**Figure 46****.** Luminosity and chromaticity coordinates of formula CR002 throughout the stability test (time (T) in days).
**Figure 47****.** Variation in pH of formulation CR002 over time (days).
**Figure 48****.** Apparent viscosity of formulation CR002 with aqueous extract.

### DETAILED DESCRIPTION

The present disclosure relates to a cork powder and to an aqueous extract thereof, as well as to a method for obtaining said powder and said extract. Furthermore, the present application relates to the use of the extract in cosmetics or medicine.

In the prior art, color can be measured by various methods. In the present disclosure, color was measured using the *CIELAB* system (or CIE *L*a*b**). *L** is the luminosity value (which defines black as 0 and white as 100), the a* chromaticity coordinate corresponds to green-red colors (where negative values correspond to green and positive values to red) and the b* chromaticity coordinate corresponds to blue-yellow colors (where negative values correspond to blue and positive values to yellow).

Measuring the particle size of cork powder can be done in various ways. In this disclosure, particle size measurement was carried out with the laser diffraction method using Mastersizer 3000 equipment, or through the standardized method of particle size analysis by mechanical sieving. In particular, with regard to the particle sizes obtained, sieves with mesh openings of 1000 µm, 800 µm, 180 µm, 63 µm and/or 16 µm were used.

### Valorization of cork powder for cosmetic use thereof

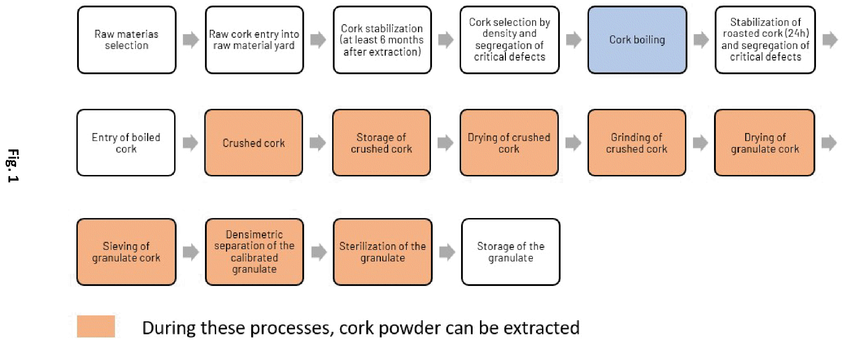

A study was carried out in order to identify which of the cork processing stages should be used to collect the cork powder, as well as the methodologies needed to treat it, with a view to its recovery as a cosmetic ingredient **(****Figure 1****).**

### Collection of cork powder at different extraction points

The identification of which stage of cork processing the cork powder intended for the new cosmetic product should be collected from was carried out. In fact, the powder was collected in the stages of crushing, drying of the crushed and granulated material, dedusting and transport of the granulated material and, finally, in the stage of sterilization of the granulated material.

### Treatment of cork powder with a view to its recovery

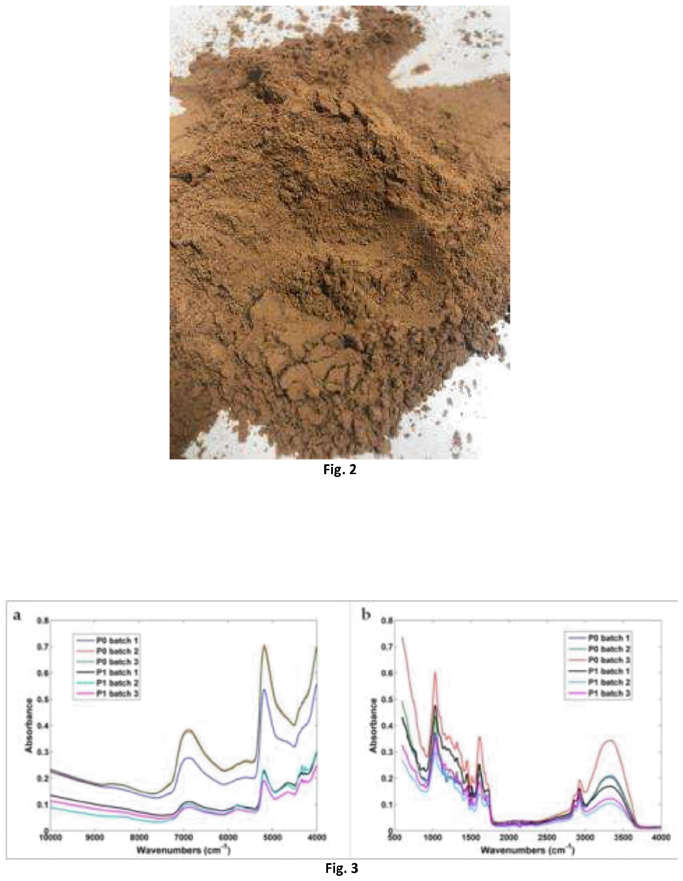

The processing of the cork was adapted to the characteristics that were needed to be guaranteed to the extracted powder. Thus, the following processes were carried out:
- **Crushing process:** shredding of cork planks and waste to obtain pieces between 2 and 35 mm. In this process, powder between 0 and 2 mm was obtained, most of which was made up of the cork coast. PG cork powder, hereinafter referred to as P0, was obtained at this stage of the process. There was heterogeneity in both the color of the grains and the dimensions of the P0 powder. The density and humidity were analyzed, with a value of 287 kg/m³ and 40%, respectively. TCA was also determined, with a value of 13.7 ng/L.
   **Drying process for the crushed material:** The drying process for the crushed material consists of passing the crushed material through two rotating drums continuously for 10 minutes. Drying is carried out by radiation obtained through water vapor. During drying, the grains of powder come loose from the crushed cork and are sucked into the dryer's dedusting filter. PS cork powder was obtained at this stage of the process.
- **Grinding process of the crushed cork** - **GRANULATION:** The granulation process consists of breaking up the crushed cork into grains between 0.2 and 8 mm in size. In this process, powder between 0 and 0.2 mm was created. The cork powder PT, hereinafter referred to as P1, was obtained at this stage of the process **(****Figure 2****),** more specifically at the final stage of the process, when the granules were separated into different grain sizes (powder from granulometric separations).

### Cosmetic formulations containing cork powder

Formulations of cosmetic make-up products and products with photoprotective capacity were developed using cork powder, in order to demonstrate the cosmetic applicability of this natural ingredient.

### Pre-formulation and formulation of cosmetic make-up and photoprotective products based on cork powder

Characterization studies were carried out on the cork powder, which included the following tests: moisture content, oil absorption, color and particle diameter.

To assess the moisture content, an infrared balance was used, where the samples were subjected to a temperature of 80 °C for 10 minutes. To determine the apparent density, a 250 ml measuring cylinder was used, into which an appropriate mass of powder sample was placed. Triplicate tests were carried out on each sample without beating/compaction.

To determine the color of the powder samples, a Chromameter CR-400 colorimeter was used, where tests were carried out for each of the powder samples, in triplicate, under the following conditions:
- Observer's degree: 2 degrees
- Primary light source: D65

The samples were analyzed in small plastic Petri dishes.

Particle diameter was determined using Malvern's Mastersizer 3000 equipment. The samples were previously prepared for analysis in the equipment.

In addition, the profile of volatile compounds that give the powders their characteristic odor was characterized using HS-SPME **(Table 1).** This test enabled the detection and identification of 10 compounds within the classes of ketones, aromatic aldehydes, monoterpenes and alcohols, allowing to assess possible differences and similarities between the profiles of the P0 and P1 powders. The semi-quantitative analysis revealed a similar profile of volatile compounds in the P0 and P1 powders. The P0 powder contained phenylethyl alcohol, which was not present in the P1 powder, as well as a slightly high concentration of pent-2-one, 4-methylpent-2-one, hex-2-one and vanillin. Limonene was only detected in P1 powder. Camphene and borneol were present in P1 powder in slightly higher concentrations and eucalyptol and camphor at much higher concentrations than in P0 powder.

**Table 1 - Analysis of the volatile compounds in the P0 and P1 powders.**

| **Compounds** | **RT(min)** | ***m*/*z*** | **R Match** | **Area (AU)** | |
|---|---|---|---|---|---|
| | | | | **PO ± SD (10⁶)** | **P1 ± SD (10⁶)** |
| Ketones | | | | | |
| Pent-2-one ^{L1} | 3.60 | 58, 71, 86 | 895 | 21.60 ± 5.72 | 19.00± 6.25 |
| 4-methylpent-2-one ^{L2} | 4.43 | 58, 85, 100 | B05 | 33.60 ± 7.51 | 23.90 ± 14.70 |
| Hex-2-one ^{L1} | 5.44 | 58, 85, 100 | 869 | 6.07 ± 0.98 | 4.81 ± 1.19 |

| Aromatic aldehydes | | | | | |
|---|---|---|---|---|---|
| Vanillin ^{L1} | 9.66 | 193, 209 | 686 | 18.50 ± 1.48 | 15.40 ± 2.22 |
| Monoterpenes | | | | | |
| Camphene ^{L2} | 9.83 | 93, 121 | 925 | 1.19 ± 0.04 | 2.74 ±. 0.71 |
| Limonene ^{L1} | 12.25 | 67, 68. 93 | B71 | --- | 2.32 ± 0.68 |
| Eucalyptol ^{L1} | 12.35 | 81, 93, 108 | 800 | 2.37 ± 1.45 | 46.40 ± 8 59 |
| Camphor ^{L1} | 15.87 | 95, 109 | 901 | 22.30 ± 0.81 | 62.00 ± 1.50 |
| Borneol ^{L2} | 16.61 | 95, 100 | 809 | 2.02 ± 0.15 | 7.18 ± 0.60 |

| Alcohols | | | | | |
|---|---|---|---|---|---|
| Phenylethyl alcohol ^{L1} | 15 | 91, 92 | 824 | 0.97 ± 0.09 | --- |

Analysis of the volatile compound profile of P0 and P1 cork powders revealed their potential as a sustainable source for obtaining fragrances and, consequently, contributing to a pleasant scent in the formulations to which they are added. Surprisingly, the P1 powder proved to be a preferable source of volatile compounds, with a higher monoterpene content than the P0 powder. Through HS-SPME analysis, it was possible to determine the volatile compounds that contribute to the organoleptic properties of the powders. While the P0 powder showed slightly higher levels of vanillin, the P1 powder showed much higher amounts of monoterpenes in the HS-SPME analysis.

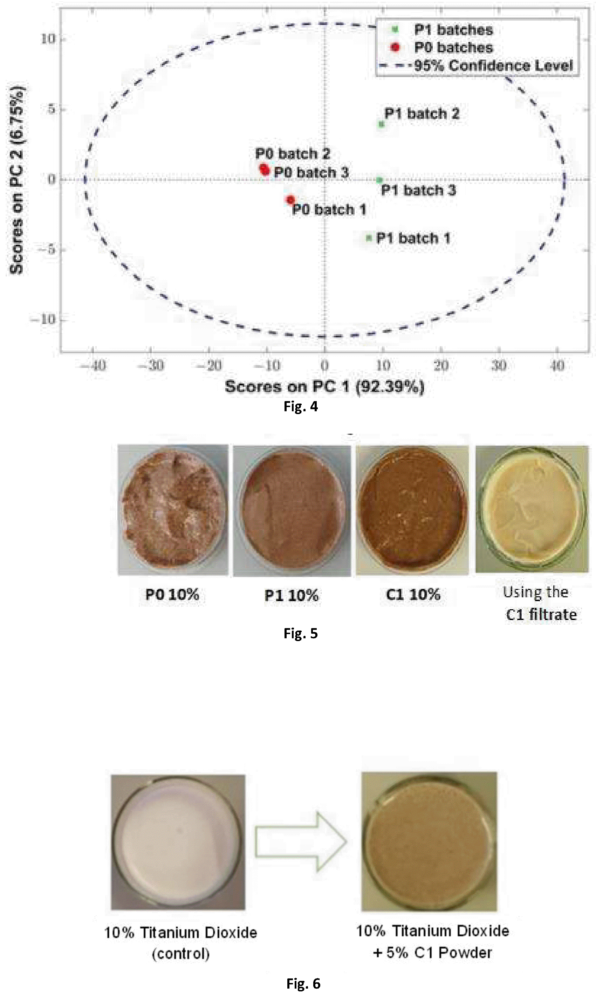

In order to analyze changes in the chemical profile of 3 batches of P0 and P1 powders, NIR spectra were obtained **(****Figure 3****).** The results revealed that the P0 batches were very similar to each other, as was also seen in the P1 batches. The spectra of the P0 powder batches showed more intense bands in the 7000 to 5200 cm⁻¹ range (water band region), which led to the conclusion that the P0 powder contains more water than the P1 powder. However, analysis of the raw NIR spectra of the samples was not enough to guarantee chemical similarity between the samples, since many different chemical bonds could absorb in the same wavenumber region. A PCA was therefore carried out to verify the difference between the P0 and P1 powders. Analysis of the PCA results **(****Figure 4****)** using the NIR spectra data revealed that cork powder samples tend to form groups according to the type of sample. Samples from the P0 batches scored negatively in principal component 1 (PC 1), while samples from the P1 batches scored positively. This reinforces the fact that the NIR spectra of the P0 and P1 powders are different, while the samples from each batch are similar.

The "P1 powder" was selected for further study, as it had the most favorable characteristics for use as a cosmetic pigment. A reproducibility study was therefore carried out on this powder to see if the same by-product produced on different days had the same characteristics, and tests were carried out on moisture content, color and particle diameter. For a more extensive characterization of the P1 powder, the following tests were carried out: pH determination, compressibility, oil absorption capacity, heavy metal content and microbiological quality.

Tests on samples of P1 powder from different days were carried out to check the reproducibility of the batches. The variability of the different batches was less than 15%, confirming adequate reproducibility **(Table 2).**

**Table 2 - Batch reproducibility assessment tests for P1 powder**

| **Parameter** | **P1 (Day 1)** | **P1 (Day 2)** | **Coefficient of variation (%)** |
|---|---|---|---|
| **Moisture content (%)** | 10.9± 2.1 | 9.9± 0.9 | 7 |
| **Color** | L*= 53.73± 0.00 | L*= 53.37± 0.07 | 1 |
| | a*= 9.06± 0.01 | a*= 8.87± 0.03 | 1 |
| | b*= 8.98± 0.01 | b*= 8.68± 0.02 | 2 |
| **Particle diameter (µm)** | 27.1± 0.1 | 22.5± 0.1 | 13 |

For the selected P1 powder, additional tests were carried out to valorize this by-product, namely the determination of pH, flow and compaction properties, oil absorption capacity, heavy metal content and microbiological quality.

To determine the pH, a 10 wt % aqueous dispersion of P1 powder was prepared under magnetic stirring for 10 minutes. The suspension obtained was filtered through a paper filter and the pH was measured in the filtrate using an HI 2211 pH/ORP/°C meter (Hanna Instruments, Woonsocket, RI, USA). The measurements were taken in triplicate **(Table 3).**

**Table 3 - Determining the pH of P1 powder.**

| **Parameter** | **Result** |
|---|---|
| **pH** | 5.47± 0.01 |

The European Pharmacopoeia 10 test and a TA.XT.plus texturometer from Stable Micro Systems were used to determine the flow properties, namely the compressibility and resistance of the compact obtained with P1 powder **(Table 4).**

**Table 4 - Determination of the compressibility index of P1 powder**

| **Parameter** | **Result** |
|---|---|
| **Compressibility Index** | 27.6 |

The oil absorption capacity of the P1 powder was studied in accordance with the ISO 787/5- 1980 standard. 1g of P1 powder was placed on a glass plate. Raw linseed oil was added drop by drop to the P1 powder. After each addition, the oil was completely incorporated into the P1 powder with a metal spatula until it spread without cracking or crumbling. The same process was carried out on 1g of kaolin, often used in cosmetic formulations for its oil absorption capacity. The weight of linseed oil that must be absorbed by a given weight of cork powder is defined as the comparative oil absorption value **(Table 5).**

**Table 5 - Determination of the oil absorption value of P1 powder, compared to the control.**

| **Control (Kaolin)** | **P1** |
|---|---|
| **37.02 g/ 100 g** | 222.74 g/ 100 g |

To determine the heavy metal content, the P1 powder samples were solubilized by microwave assisted closed-vessel acid digestion in an ETHOS EASY microwave oven equipped with an SK-15 easyTEMP high-pressure rotor, following a procedure based on US Environmental Protection Agency (EPA) method 3052 (microwave-assisted acid digestion of siliceous and organic-based matrices). Blank samples were obtained using the same procedure. For analytical quality control purposes and considering the type of sample analyzed in this study, two certified reference materials (CRM) were used: Cabbage Powder (BCR-679) and Bladderwrack (Fucus vesiculosus) Powder (ERM-CD200), both from the Joint Research Centre of the European Commission, subjected to the same sample pretreatment. The samples were analyzed in triplicate and a blank test was carried out on each digestion batch. Trace elements were determined using inductively coupled plasma mass spectrometry (ICP-MS) **(Table 6).**

**Table 6 - Determination of heavy metal content (mean± SD) in P1(ppm) n=3**

| **Heavy metals** | **P1** |
|---|---|
| **As** | 0.17± 0.03 |
| **Cd** | 0.06± 0.00 |
| **Hg** | 0.01± 0.00 |
| **Pb** | 1.20± 0.04 |

In order to optimize the characteristics of the P1 powder, which proved to be the most promising, its processing was carried out. To do this, the P1 powder was sieved using sieves with mesh openings of 180 µm and 63 µm. The fraction above 63 µm (fraction E) was collected to obtain bioactive extracts and the fraction below 63 µm (fraction C) was used to obtain make-up pigments and carry out pre-formulation studies on this type of product.

Subsequently, the C fraction was also processed in order to obtain a more homogeneous powder with a smaller particle diameter. Firstly, part of the C fraction (25g) was suspended in 400 mL of water using magnetic stirring (1250 rpm) for 20 minutes. The suspension was then filtered through a G4 crucible filter with a pore size of 16 µm. The fraction retained on the surface of the filter is collected and subjected to a drying process in a fluid bed oven at 50 °C for 1 hour, resulting in the C1 fraction, with a particle diameter of between 16 and 63 µm. To avoid waste, the filtrate obtained is also used for incorporation into semi solid formulations.

The C1 fraction is the powder fraction that was used for the pre-formulation studies as a pigment for make-up products. This fraction was extensively characterized using tests such as moisture content, particle diameter, apparent density, pH, color, oil absorption, hygroscopy, water soluble content and volatile matter at 105°C **(Table 9).**

Hygroscopy was assessed in accordance with the European Pharmacopoeia 10. The weighing container and stopper were weighed (m₁). In summary, 1.5g of C1 powder was placed in the bottom of the weighing container, which was subsequently stopped and weighed (m₂). The unperforated weighing container was placed in a desiccator containing a saturated solution of ammonium sulphate (relative humidity = 78.6%) at 25 °C for 24 hours. The weighing container was stopped and weighed (m₃).

The water-soluble content of C1 powder was studied in accordance with ISO 787-3:2000. 5g of C1 powder (m₀) was suspended in 200mL of boiling water and stirred for 5 minutes. The mixture was then transferred to a 250 mL volumetric flask and diluted to the mark with water. The filtrate was placed in a porcelain dish and the residue was dried in an oven at 105°C for 2 hours. The process was repeated until two consecutive weighing did not differ by more than 10% (m₁).

The volatile matter at 105 ° C of C1 powder was studied in accordance with ISO 787-2:2021. 10 g of C1 powder were placed at the bottom of the weighing flask in a uniform layer. The weighing flask was placed in an oven at 105 °C for one hour. The process was repeated until two.

The microbiological quality of the P1 powder and C1 fraction was then assessed. In this context, it should be noted that the microbiological quality analysis protocol was prepared in accordance with ISO 21148:2017 and European Pharmacopoeia 10, specifically the chapter "Microbiological examination of non-sterile products". The sample was prepared by diluting 1 g of P1 powder in 10mL of saline solution. Serial dilutions up to 10⁻³ were also prepared. Aliquots of 100 µL of the original samples and dilutions were spread, in duplicate, on the surface of Tryptic Soy Agar (TSA) medium for isolating bacteria, and incubated for 24-48 hours at 30-35 ± 2°C. Bacterial isolates were then identified using the Gram stain method. This was followed by the detection of specific microorganisms, which should be absent in the sample studied - *E. coli, S. aureus, P. aeruginosa* and C. *albicans,* in the respective selective media, according to a protocol defined on the basis of ISO standards and the European Pharmacopoeia 10. In order to identify the Enterobacteriaceae present in the P1 powder sample according to their biochemical characteristics, an API (Analytical Profile Index) 20 E was carried out. An ID 32 C was also carried out to identify the yeasts present in the P1 powder sample according to their biochemical characteristics.

**Table 7 - Determining the microbiological quality of P1 powders and C1 fraction by counting total aerobic microorganisms and identifying specific microorganisms.**

| | **P1** | **C1** |
|---|---|---|
| ***Total Aerobic Microorganism Count*** | 4.7 x 10⁴ CFU/g | 8.5×10⁴ CFU/g |
| ***Escherichia coli*** | Absent in 1g | Absent in 1g |
| ***Pseudomonas aeruginosa*** | Absent in 1g | Absent in 1g |
| ***Staphylococcus aureus*** | Absent in 1g | Absent in 1g |
| ***Candida albicans*** | Absent in 1g | Absent in 1g |

Even so, as the total count of aerobic microorganisms exceeded the maximum limit defined in ISO 17516:2014 and European Pharmacopoeia 10 **(Table 7),** the C1 powder was heat-treated or chemically treated under different conditions in order to find out which less aggressive conditions would allow the microbiological quality of the ingredient to be optimized to a value within the regulatory limits (less than 1.00x10³ CFU/g). Various methods were implemented with the aim of reducing the microbiological load of C1 powder - **Table 8.**

**Table 8 - Optimization of the microbiological quality of C1 powder**

| **Conditions** | **Total aerobic microorganism count** (after 48 hours of incubation in an oven at 25°C) |
|---|---|
| 15% hydroalcoholic mixture | 6.3x10⁴CFU/g |
| Sterilization at 180°C for 2 hours | 0 CFU/g |
| Sterilization at 180°C for 1 hour | 0 CFU/g |
| Sterilization at 180°C for 30 min | 0 CFU/g |
| Sterilization at 180°C for 15 min | 3.1x10³ CFU/g |

It was found that changing the processing of C1 powder by suspending it in a 15% hydroalcoholic mixture instead of water was not effective in reducing the microbiological load. On the other hand, sterilizing C1 powder at 180°C for 2 hours, 1 hour and 30 minutes completely eliminated the microbiological load present. Despite a considerable reduction in the microbiological load, the sterilization process at 180°C for 15 minutes did not ensure the required microbiological quality. In this sense, the least aggressive conditions that improve microbiological quality to a value below 1.00x10³ CFU/g correspond to heating the C1 powder at 180°C for 30 minutes. This sterilized C1 powder was given the designation C1E.

### Characterization of C1E Powder

Next, the sterilized C1 powder (C1E) (obtained by heating the C1 powder at 180°C for 30 minutes) was characterized. This is because, the process for obtaining C1 powder had been altered by adding a 180°C heating step (C1E), it was necessary to carry out a new battery of physicochemical characterization tests on the powder to check for any significant changes **(Table 9).** The tests on the C1 powder (non-sterilized) were repeated when analyzing the C1E powder, so that the values obtained in each test could be directly compared.

**Table 9 - Physicochemical characterization tests of C1E powder, compared to C1 powder (non sterilized)**

| **Test** | **C1** | **C1E** |
|---|---|---|
| *Moisture content* (% - loss on drying) | 6.12± 0.16 % | 3.03± 0.42 % |
| *Particle diameter* (DX50 µm - Laser Diffraction) | 30.7± 1.5 µm | 35.5± 0.28µm |
| *pH* (Potentiometry) | 6.04±0.06 | 6.21±0.08 |
| *Color* (L*a*b* - Reflectometry) | L* = 48.63±0.48 | L* = 47.73±0.07 |
| | a*= 8.03±0.00 | a*= 8.22±0.04 |
| | b*= 15.09±0.00 | b*= 14.20±0.11 |
| *Oil absorption* (g/ 100 g - ISO 787/5-1980) | ≈ 5 times higher to kaolin | ≈ 5 times higher to kaolin |
| *Heavy metal content (ppm)* | As< 3 ppm | As< 3 ppm |
| | Cd< 1 ppm | Cd< 1 ppm |
| | Hg< 1 ppm | Hg < 1 ppm |
| | Pb< 10 ppm | Pb< 10 ppm |
| *Hygroscopy* (% - European Pharmacopoeia 10) | 1.46± 0.49 % | 9.04± 0.08 % |

Thus, no marked changes between the parameters analyzed in the C1 powder and the C1E powder were verified. The results for moisture content and hygroscopy showed the most disparate values. The C1E powder had half the moisture content and a hygroscopic value approximately 6 times higher than the C1 powder. This is due to the fact that the C1E powder was subjected to an extreme heating process, forcing the water incorporated in the sample to evaporate.

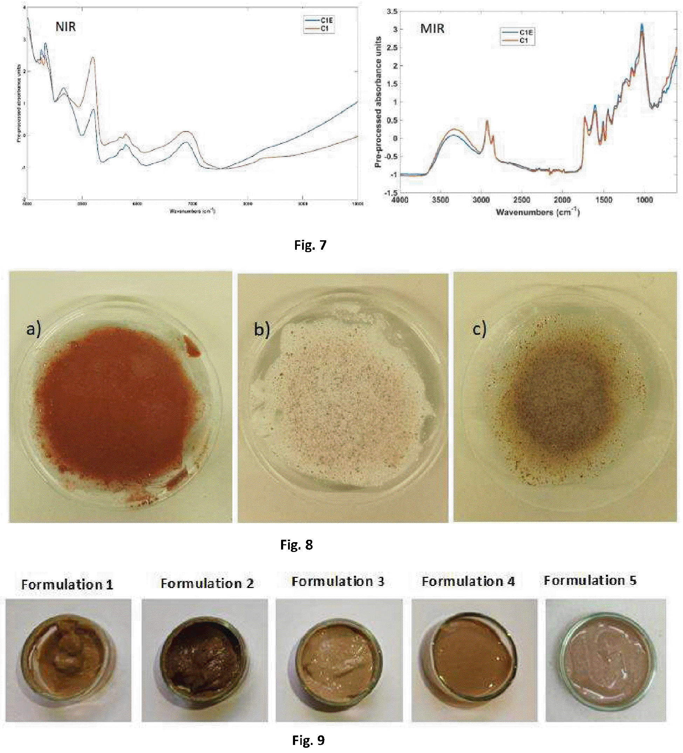

In addition, in order to be able to analyze the changes in the chemical profile of C1 powder that may have been induced by the thermal process to which it was subjected, C1 powder from a previous batch and C1E powder were analyzed using spectroscopic methods - near infrared (NIR) and mid infrared (MIR) spectroscopy **(****Figure 7****).** NIR makes it possible to explore the physical and chemical characteristics of solid samples, exploring the interactions between matter and light in a non-destructive way and requiring only a small amount of C1E. The spectra recorded in the NIR region (10 000-4 000 cm⁻¹) are sensitive to a wide variety of compounds and molecular interactions. In the MIR, most functional groups can be observed, since their vibrational resonances lie in this range of excitation frequencies and even complicated and similar structures, such as polysaccharides, can be differentiated. The MIR complements the NIR and covers the 4000-400 cm⁻¹ region and contains information on fundamental molecular vibrations. In hydrated samples, the NIR spectra show two primary water absorption bands at frequencies of ^{~}5200 and 7000 cm⁻¹. Comparing the NIR and MIR graphs of the C1 and C1E powders, it is possible to perceive that there are differences due to the greater presence of water in the C1 powder than in the C1E powder **(****Figure 7****).** In other words, there is no change in the compounds present (since both graphs show the same peaks), but rather in the intensity of their bands.

Another of the studies carried out was with dispersant liquids used in cosmetics, in an attempt to facilitate the dispersion of powder in semi-solid or fluid formulations. Thirteen liquids commonly used to disperse pigments in cosmetics were selected (propylene glycol, glycerin, castor oil, sweet almond oil, liquid paraffin, Crodamol IPIS, Ceraphyl 791, 1,3 Butylene glycol, Hallbrite BHB, Evertouch TM, Dimethicone copolyol, 96° alcohol, Cyclomethicone ne tetramera) and then mixed with C1 powder. The mixtures were left to stand and after 24 hours their appearance was assessed to see which mixture was the most homogeneous. The liquids chosen were propylene glycol and glycerin.

For the pre-formulation studies, the by-products were first incorporated into a semi-solid formulation, namely an O/W emulsion. Powders P0, P1 and C1 were tested, as well as the filtrate resulting from processing P1 powder. The appearance of the formulations is shown in **Figure 5****.**

In addition, the Sun Protection Factor (SPF) enhancing capacity of C1E powder in semi-solid formulations known as anti-solar emulsion **(Table 10)** was evaluated using Labsphere's UV-2000S Sunscreen Analyzer.

**Table 10: Sunscreen formulation.**

| **Phase** | **Ingredients** | **Amount (wt%)** | |
|---|---|---|---|
| | | **F1** | **F2** |
| **I** | Cutina MD^{®} _{(mixture of Glyceryl Monostearate, Cetostearyl Alcohol, Cetyl Palmitate and Coco-Glycerides)} | 4.4 | 5.4 |
| I | Eumulgin^{®} B-1 _{(Ceteareth-12 (ethoxylated cetostearyl alcohol))} | 0.4 | 1.4 |
| I | Mygliol^{®} 318 _{(medium chain triglycerides)} | 7.4 | 8.4 |
| I | Cetiol^{®} SN _{(cetearyl isononanoate)} | 7.4 | 8.4 |
| I | Titanium dioxide | 10.00 | 10.00 |
| I | C1E powder | --- | 5.00 |
| II | Water | Qs 100 | |
| **Procedure:** | | | |
| 1. Heat the ingredients of phase I to 70 °C; | | | |
| 2. Heat the water to 70 °C. | | | |
| 3. Add Phase I to Phase II in a high shear mixer. Start cooling. | | | |
| 4. Mix the ingredients from Phase B and add to the main container. | | | |
| 5. Cool to 40 °C under stirring and then homogenize in a homogenizer in a Unguator. ^{®} (mixing system). | | | |

C1E powder was incorporated at 5 wt% into a formulation containing 10 wt % titanium dioxide, in which the SPF was determined. At the same time, the SPF of the same formulation with only 10 wt % titanium dioxide, considered the control, was determined. The formulation with C1E had an SPF value around 2.5 times higher than the control, confirming the SPF-promoting capacity of C1 powder **(****Figure 6****):**
- Sunscreen with 10 wt % titanium dioxide: 25.30±2.54 (mean ±SD)
- Sunscreen with 10 wt % titanium dioxide with 5 wt% C1 powder:
   63.67±13.82(mean ±SD) (2.5X higher than control)

### Pre-formulation study of C1E powder

Following on from the pre-formulation study previously started for all the by-products of the cork industry (P0 Powder, P1 Powder and C1 Powder), the pre-formulation studies for C1E Powder followed, consisting of various tests of mixing C1E Powder with other pigments widely used in decorative cosmetic formulations, namely titanium dioxide and red iron oxide **(****Figure 8****),** at different concentrations. The aim was to obtain a relatively homogeneous, beige/brownish mixture for decorative make-up products (cream with color). The proportion of C1E powder and titanium dioxide **(****Figure 8c)** is the closest to what was desired.

Next, different mixtures of C1E powder and titanium dioxide were incorporated into different base formulations **(****Figure 9****).** Formulation 5 **(Table 11)** was the one that looked closest to what was desired and was therefore selected for further experimental formulation studies.

**Table 11 - Composition of formulation 5 (BBc05) and respective preparation procedure**

| **Phase** | **Ingredients** | **Amount (wt%)** |
|---|---|---|
| A | Olivatis^{®} 18 _{(olive oil polyglyceryl-6 esters, sodium stearoyl lactate, cetostearyl alcohol)} | 5.00 |
| A | Dicaprylic ether | 8.00 |
| A | Squalene | 3.00 |
| A | Miglyol 812 _{(caprylic/capric triglyceride)} | 2.00 |
| A | Stearic acid | 2.00 |
| A | Sweet almond oil | 2.00 |
| A | C1E powder | 2.00 |
| A | Titanium dioxide | 0.40 |
| B | Xanthan gum | 0.20 |
| B | Glycerin | 4.00 |
| B | Propanediol | 1.50 |
| C | Euxyl^{®} _{(phenoxyethanol+ Benzyl alcohol+ Ethylhexylglycerin+ Tocopherol)} | 1.50 |
| C | Water | qs 100 |
| **Procedure:** | | |
| 1. Heat the ingredients of phase A to 70 °C; | | |
| 2. Heat the water to 70 °C. | | |
| 3. Add Phase A to the water in a high shear mixer. Start cooling. | | |
| 4. Mix the ingredients from Phase B and add to the main container. | | |
| 5. Cool to 40 °C under stirring and then homogenize in a high-speed homogenizer. | | |

Formulation studies were performed with C1E powder (cream with color), involving the following tests on the base formulation: Mechanical Stress Test, Rheological Characterization and Spreadability.

### Mechanical stress test

The centrifugation test subjects emulsions and suspensions to extreme gravitational forces with the purpose to assess the stability of the formulation by accelerating possible phase separation and sedimentation effects. Thus, before embarking on a longer stability study, this test makes it possible to understand whether the base formulation is in fact physically stable. If there is phase separation, it means that the cream is not stable and therefore the formulation will have to be changed before proceeding to the other tests.

In general, the stress test conditions - RPMs - and centrifugation time vary depending on the study. However, the most common RPMs for cream centrifugation stress tests are 3000-5000 RPMs, and the most common centrifugation time is 30 minutes. Typical centrifugation settings are 3500 RPMs for 30 minutes. In this sense, the selected base formulation was subjected to 3500 RPMs for 30 minutes at 25°C. There was no appearance of two distinct phases, i.e. the formulation did not show phase separation under the test conditions, demonstrating its stability.

### Rheological characterization of the base formulation

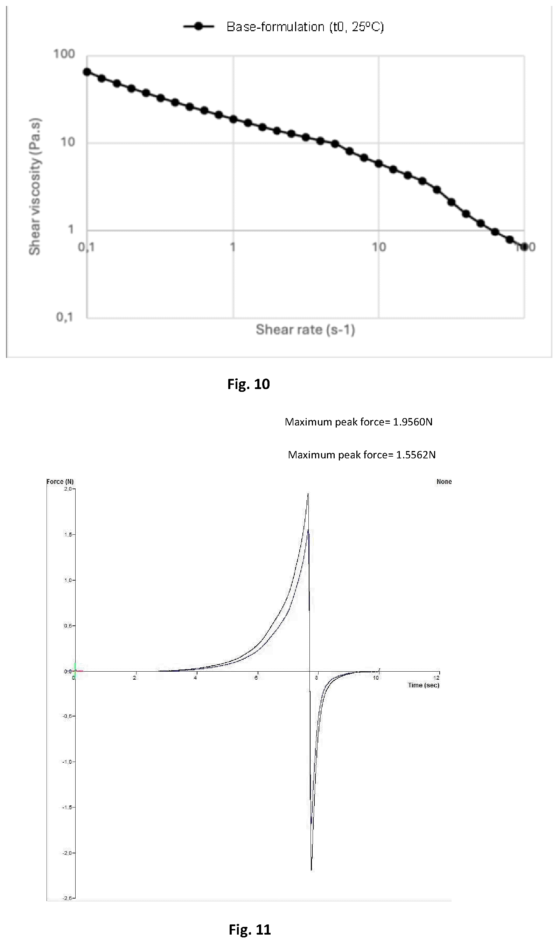

In this context, base formulations were selected in order to study the stability of the ingredients developed when incorporated into cosmetic product formulations. Taking into account the purpose of the ingredient, a make-up base formulation was selected (where the C1 powder fraction was to be incorporated). The rheological behavior of the make-up base formulation was studied, namely flow behavior. The formulation showed non-Newtonian fluid behavior of the pseudoplastic (shear thinning) type **(****Figure 10****),** i.e. the apparent viscosity decreased with increasing shear rate.

The behavior exhibited by the base formulation over time is confirmed when the power law model is applied. The negative value of "n" indicates that the sample is more viscous at low shear rates, and the apparent viscosity decreases as the shear rate increases **(Table 12).**

**Table 12: Application of the power law model.**

| Base formulation (25°C) | |
|---|---|
| **K** | 18.599±0.092 |
| **n** | -0.510±0.022 |
| **R²** | 0.997±0.001 |

In order to study the physicochemical stability and activity of the ingredients over time, the respective stability assessment protocols were also developed during this period, both for the ingredient (C1E fraction) and for the formulation.

### Spreadability

To assess the spreadability of the cream, a texturometer was used, in which a cone-shaped probe was attached and, as a base, a probe also in the shape of a cone, into which the sample to be analyzed was introduced. The probe moves downwards at a set speed and distance.

The ease with which the conical probe moves in the cream is reflected in the product's spreading capacity. As soon as the probe comes into contact with the test sample, the force measured increases steadily until it reaches a maximum value, then the probe reverses direction and withdraws from the sample. In this case, the measured force is negative because the formulation adheres to the probe, resisting its movement out of the sample container. It is possible to understand that storage temperature does not contribute to marked differences between the spreadability of the C1E powder formulation analyzed **(****Figure 11****).**

The C1E powder, due to its properties (low moisture content; adequate particle diameter; color; microbiological quality), was selected for pre-formulation studies (powder mixtures with pigments at different concentrations, dispersion in different solvents, among others) and formulation studies (stress test, rheological characterization, among other parameters) using C1E powder as an ingredient, selecting a base formulation suitable for its incorporation, as well as the quantity of powder to be used.

### In vitro evaluation of the toxicity of cork powder

The toxicity of cork-derived powders, namely samples P0, P1 and fraction C1, was assessed on a representative skin cell line (human keratinocytes). Cell viability was determined after the cells were exposed to different concentrations (100, 50, 25, 10 and 5 mg/mL) of each of the samples.

It was possible to verify that cell viability approached 90% when the cells were exposed to samples of cork powder at the following maximum concentrations: P0= 50 mg/mL (95% viability); P1 = 10 mg/mL (94% viability) and C1 = 25 mg/mL (93% viability).

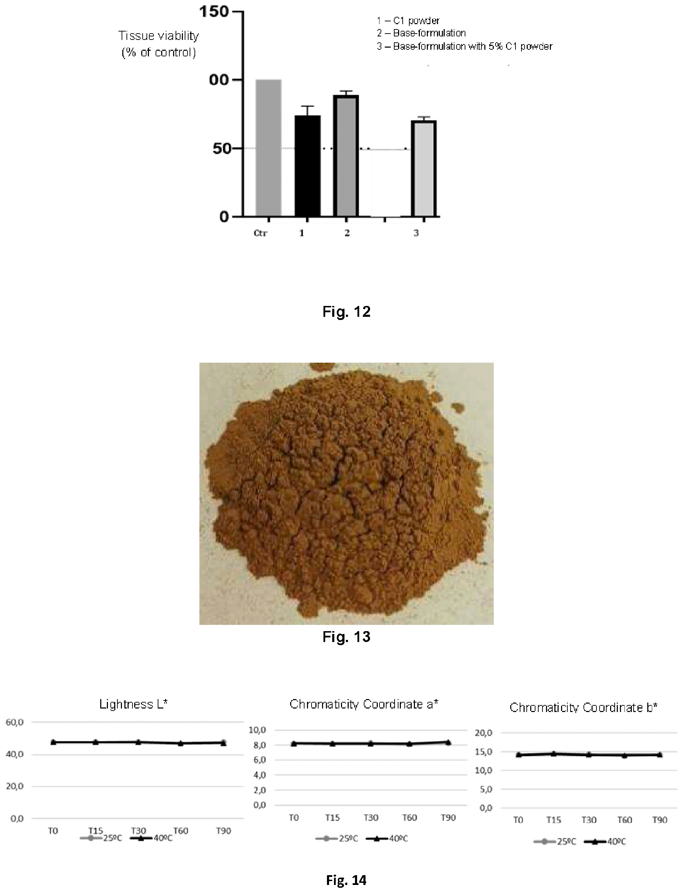

Finally, the irritant potential of the selected cork powder was assessed using a 3D model, Episkin, in accordance with OECD Test *Guideline* 439. Briefly, the tissues were exposed to C1E powder, the base formulation (formulation without C1 powder) and the base formulation with 5% C1 powder **(****Figure 12****).** Tissue viability was determined using the MTT test. According to this skin irritation test, if the average tissue viability is equal to or less than 50%, the analyte tested is considered irritating, which was not the case. C1 powder can therefore be considered safe for use, as can the formulation tested, incorporating C1 powder at a concentration of 5 wt% **(****Figure 12****).**

### Physical stability studies and characterization of quality parameters

### C1E powder stability study

In order to study the physicochemical stability and activity of the ingredient over time, a stability assessment protocol was developed for C1E powder **(Table 13).**

**Table 13 - Summary of the C1E powder stability protocol**

| **Test** | **TO** | | **T15** | | **T30** | | **T60** | | **T90** | |
|---|---|---|---|---|---|---|---|---|---|---|
| | 40 °C 75% RH | 25 °C | 40 °C 75% RH | 25 °C | 40 °C 75% RH | 25 °C | 40 °C 75% RH | 25 °C | 40 °C 75% RH | 25 °C |
| **Appearance** | X | X | X | X | X | X | X | X | X | X |
| **Color** | X | X | X | X | X | X | X | X | X | X |
| **Heavy metals** | | X | | | | | | | | |
| **Particle size** | | X | | | | | | | | X |
| **Particle morphology** | | X | | | | | | | | X |
| **pH** | X | X | X | X | X | X | X | X | X | X |
| **Moisture content** | X | X | X | X | X | X | X | X | X | X |
| **Odor** | X | X | X | X | X | X | X | X | X | X |
| **FTIR** | X | X | X | X | X | X | X | X | X | X |
| **NIR** | X | X | X | X | X | X | X | X | X | X |
| **Microbiological quality** | | X | | | | | | | | X |

| | | | | | | | | | | |
|---|---|---|---|---|---|---|---|---|---|---|
| X: Test carried out | | | | | | | | | | |

The powder stability study took place over 90 days and was stored at two different temperatures: 25°C and 40°C. The storage container consisted of a PVP bag with a zipper closure system, which came into direct contact with the powder and was placed inside a plastic container with two lids, reducing the powder's contact with the outside environment as much as possible. Various physical, chemical and microbiological parameters were analyzed throughout the analysis period.

The photographic records taken during the stability study concluded that, macroscopically, there were no changes in color or homogeneity. The odor of the C1E powder, which is woody, remained intense and constant throughout the analysis period.

At the same time, a more objective analysis of the color of the C1E powder was carried out using a colorimeter, which provides information on the parameters of luminosity (L*) and chromaticity coordinates a* and b*. It was therefore confirmed that there were no considerable differences over the analysis time, regardless of temperature **(****Figure 14****).**

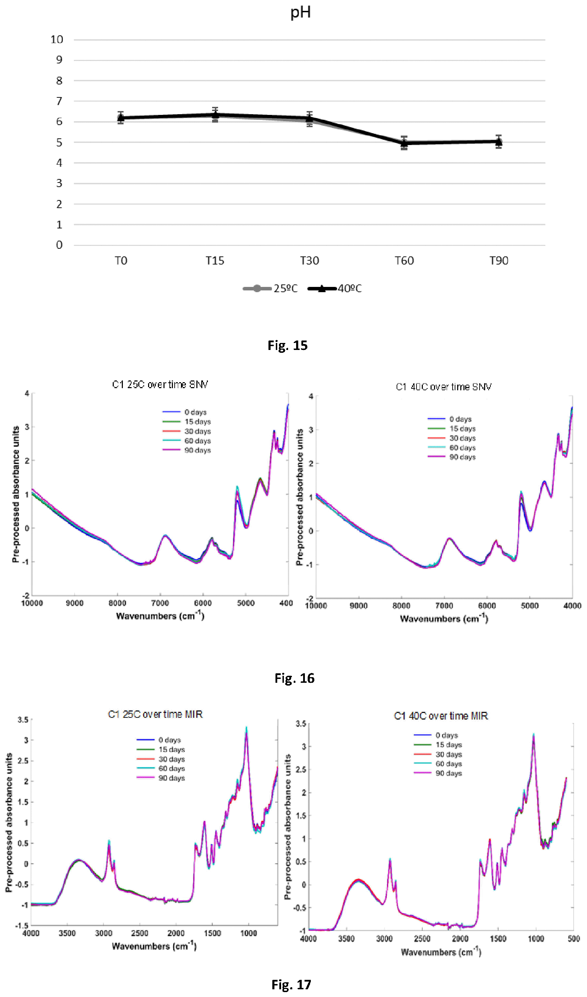

The pH remained approximately constant throughout the analysis period **(****Figure 15****).** There was a slight drop in pH from time 30 onwards, but the value of the parameter remained within the range compatible with the pH of the skin.

With regard to the spectrometric tests, there were some changes in the NIR in relation to the peaks corresponding to the ^{~}5200 and 7000 cm⁻¹ zones, which is possibly due to the fact that the C1 powder is hygroscopic and has the capacity to absorb water when the tests are carried out. The NIR and MIR graphs for each temperature remain practically constant over time, as can be seen from the almost total overlap at the different times

### (Figures 16 and 17).

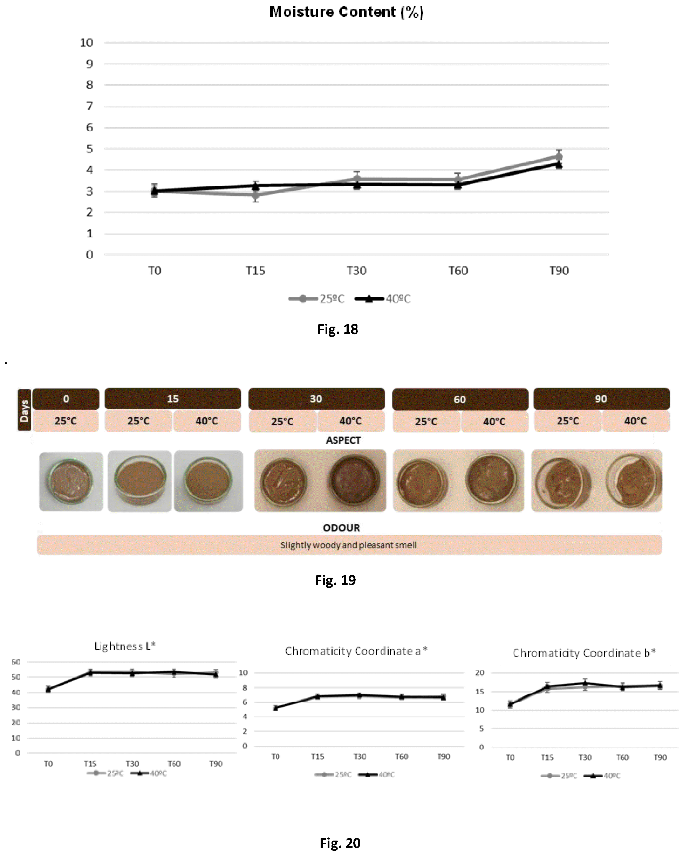

The moisture content of the C1E powder was also evaluated over the course of the 90-day test. There was a slight increase in the moisture content at time 90, which may be due to the fact that the powder had absorbed some moisture when it was weighed for the test. Even so, this parameter remained practically constant throughout the analysis period **(****Figure 18****).**

The microbiological quality was tested at the start of the trial and at day 90 after the start of the storage trial at 25°C and 40°C. At time 0, the powder was analyzed immediately after the heating process at 180°C for 30 minutes. The test was carried out inside a horizontal laminar flow chamber in order to reduce possible risks of sample contamination. The C1E powder sample analyzed (consisting of a 1:10 dilution) was prepared and properly homogenized. Serial dilutions (10⁻¹, 10⁻² and 10⁻³) were then prepared in duplicate. The total aerobic microorganism count was 0 CFU/g, 48 hours after the start of the analysis. The procedure was repeated at day 90 of the stability study, both for the sample stored at 25°C, as well as 40°C. In these cases, the count of aerobic mesophilic microorganisms was higher than that determined at time 0, but still lower than the limits established in ISO 17516:2014. Particle size and hygroscopy remained practically constant at times 0 and 90 **(Table 14).**

**Table 14. - Results at t=0 days, t=90 days (25°C) and t=90 days (40°C)**

| **Time** | t= 0 days | t=90 days | t=90 days |
|---|---|---|---|
| **Storage conditions** | N/A | 25°C | 40°C |
| **Microbiological quality** | 0 CFU/g | 1.00 x 10³CFU/g | 1.00 x 10²CFU/g |
| **Particle size** | 35.5± 0.28 µm | 34.6± 0.5 µm | 34.4± 0.2 µm |
| **Hygroscopy** | 9.04±0.67 | 8.26±0.49 | 9.24±0.48 |

### Stability studies of the C1E powder formulation

In order to study the physicochemical stability and activity of formulation 5 (BBc05) over time, a stability assessment protocol was developed for C1E powder **(Table** 15).

**Table 15: Summary of the stability protocol of the C1E powder formulation (BBc05).**

| **Test** | **TO** | **T15** | | **T30** | | **T60** | | **T90** | |
|---|---|---|---|---|---|---|---|---|---|
| | **25 °C** | **40 °C 75% RH** | **25 °C** | **40 °C 75% RH** | **25 °C** | **40 °C 75% RH** | **25 °C** | **40 °C 75% RH** | **25 °C** |
| **Appearance** | X | X | X | X | X | X | X | X | X |
| **Color** | X | X | X | X | X | X | X | X | X |
| **pH** | X | X | X | X | X | X | X | X | X |
| **Odor** | X | X | X | X | X | X | X | X | X |
| **Microbiological quality** | X | | | | | | | | X |
| **Spreadability** | X | | | | | | | | |
| **Viscosity** | X | | | | | | | X | X |
| **Thixotropy** | X | | | | | | | X | X |

| | | | | | | | | | |
|---|---|---|---|---|---|---|---|---|---|
| X: Test carried out | | | | | | | | | |

The powder stability study took place over 90 days and was stored at two different temperatures: 25 and 40°C. The storage container consisted of a glass vial with a capacity of 20 mL and a closure system consisting of a metal lid, doubly sealed with parafilm, reducing contact between the developed semi-solid formulation and the outside environment as much as possible. Various physical, chemical and microbiological parameters were analyzed throughout the analysis period.

Before carrying out the battery of tests to assess the physical, chemical and microbiological stability of the formulation with 2 wt% C1E powder, it was subjected to the mechanical stress test (at a centrifugation at 3500 RPMs for 30 minutes). It was found that, regardless of storage temperature, the formulation remained stable over time, after 90 days in both temperature conditions (25°C and 40°C).

From the photographic records taken during the stability study, it can be concluded that, macroscopically, there were no morphological changes, particularly color and homogeneity **(****Figure 19****).** The odor of the C1E powder, woody and slightly woody, remained intense and constant throughout the analysis period.

At the same time, the color of the C1E powder formulation was analyzed more objectively using a colorimeter, which provides information regarding the parameters of luminosity (L*) and chromaticity coordinates a* and b*. It was therefore confirmed that no considerable differences were detected over the analysis time, regardless of the temperature **(****Figure 20****).**

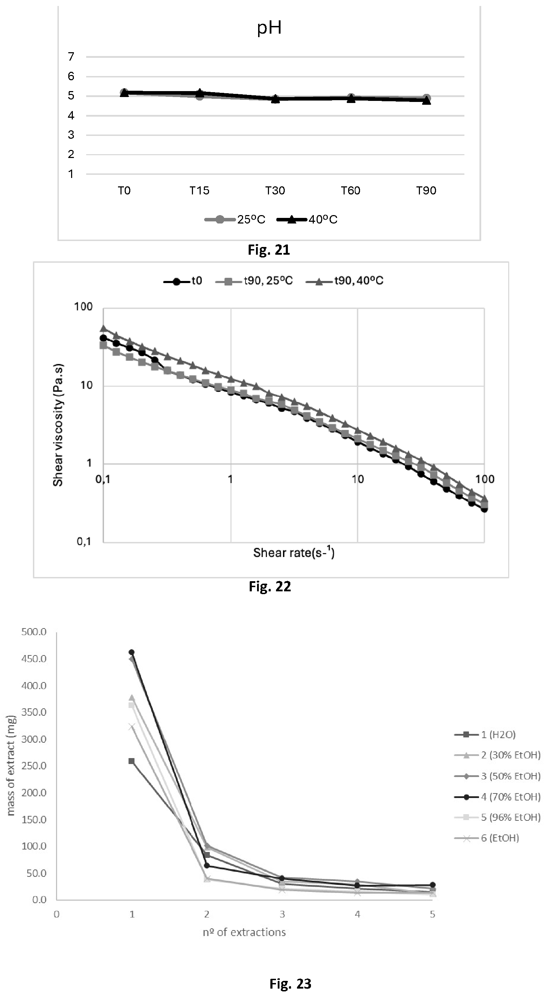

The pH value of the formulation remained approximately constant for 90 days **(****Figure 21****).**

As far as the rheological behavior of the formulation is concerned **(****Figure 22****),** as had already been seen for the base formulation, formulation 5 (BBc05) maintained a non-Newtonian fluid behavior of the pseudoplastic (shear-thinning) type.

The behavior exhibited by BBc05 cream over time (non-Newtonian fluid) is confirmed when the power law model is applied. The behavior of formulation 5 (BBc05) is the same as that described for the base formulation. The negative value of "n" indicates that the sample is more viscous at low shear rates, and the apparent viscosity decreases as the shear rate increases **(Table 16).** There was a slight reduction in the consistency coefficient, which was more significant at 40°C.

**Table 16: Parameters of the power law model (n=2).**

| | t0 (25°C) | t90 (25°C) | t90 (40°C) |
|---|---|---|---|
| *K* | 16.545±11.818 | 13.514±7.185 | 11.302±0.831 |
| *n* | -0.653±0.051 | -0.563± 0.017 | -0.633±0.012 |
| *R²* | 0.996±0.000 | 0.997± 0.000 | 0.997±0.000 |

The microbiological quality of formulation 5 (BBc05 cream) was also analyzed after preparation and at time 90, after storage at different temperatures (25°C and 40°C). In accordance with ISO 21148:2017, the formulation was initially neutralized with a mixture of 3 wt%, Tween80, 0.3 wt % Lecithin, 3 wt % saponin and 0.1 wt% histidine. The microbiological quality assessment was carried out on the samples of formulation 5 (BBc05), both in sterile serum and enriched in TSB, so that it could be checked whether there was in fact any microbiological growth during enrichment. Upon analysis of the total microbiological count, there was no growth observed under the conditions used in the test (ISO 17516:2014).

### CORK POWDER EXTRACT

### Preparation of cork powder extracts

In a preferred embodiment, the extracts were firstly obtained by treating the P1 cork powder fraction, corresponding to a finer and clearer cork powder obtained at a later stage in the production cycle of the cork industry, by heating at 40 °C with the following solvents: deionized water, 30% ethanol (v/v), 50% ethanol (v/v), 70% ethanol (v/v), 96% ethanol (v/v) and absolute ethanol. The choice of these solvents was based on the criteria of safety, compatibility with skin tissue and sustainability. In each case, 5 g of the cork powder (weighed) was mixed with 5 x 100 ml of solvent and the mixtures were stirred for 5 x 5 h on a stirring plate at 700 revolutions per minute (rpm). The extracts were filtered using a Büchner funnel with a glass fiber filter membrane (diameter 47 mm) and then concentrated at 40 °C under vacuum in the rotavapor.

After concentrated, the extracts were collected in sample holders in order to find out their mass. The results are shown in the following table **(Table 17).**

**Table 17 - Determination of extract mass**

| **Number of extractions** | **Mass of extracts (mg)** | | | | | |
|---|---|---|---|---|---|---|
| | **H2O** | **30% EtOH** | **50% EtOH** | **70% EtOH** | **96% EtOH** | **EtOH** |
| **1** | 259.5 | 378.9 | 450.3 | 462.2 | 363.2 | 323.9 |
| **2** | 83.3 | 98.1 | 100.8 | 63.7 | 37.9 | 39.3 |
| **3** | 30.0 | 34.6 | 42.0 | 39.7 | 21.1 | 18.7 |
| **4** | 21.0 | 28.6 | 34.5 | 26.5 | 15.9 | 13.3 |
| **5** | 14.8 | 11.2 | 21.2 | 28.2 | 10.6 | 13.4 |

Based on the data obtained, the 1^{st} extraction was the one that allowed the greatest quantity of compounds to be collected from the cork powder. Among these, the extracts obtained from the mixtures of 70 % (v/v) in EtOH and 50% (v/v) in EtOH were the ones with the greatest mass.

As the number of extractions increased, a decrease in the mass of the extracts was observed. From the 3^{rd} extraction onwards, the variation in the mass of the extracts was not very pronounced, which is why it was not considered profitable to carry out more than two extractions **(****Figure 23****).**

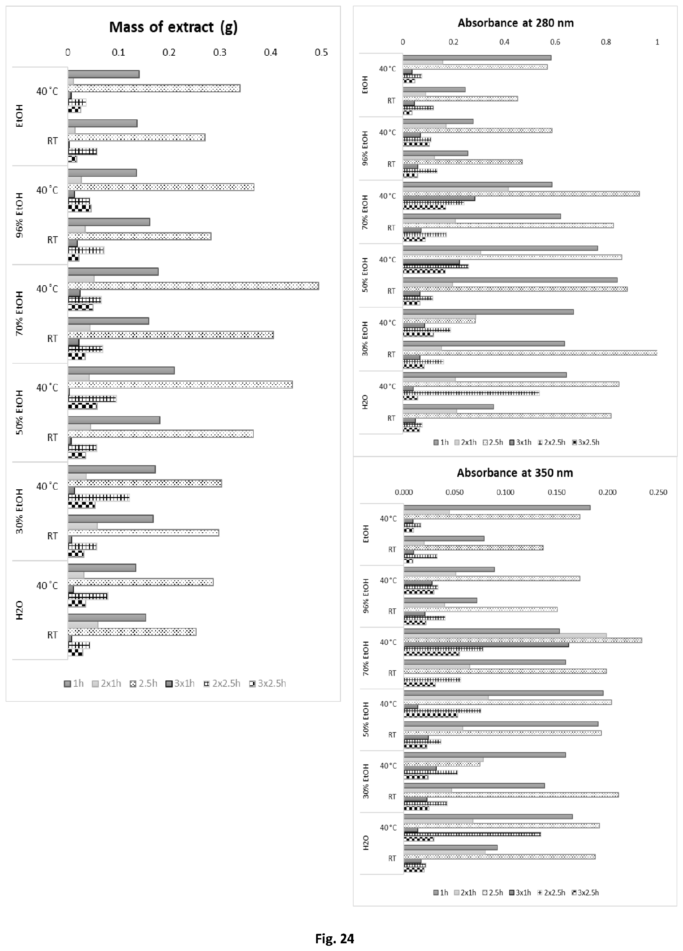

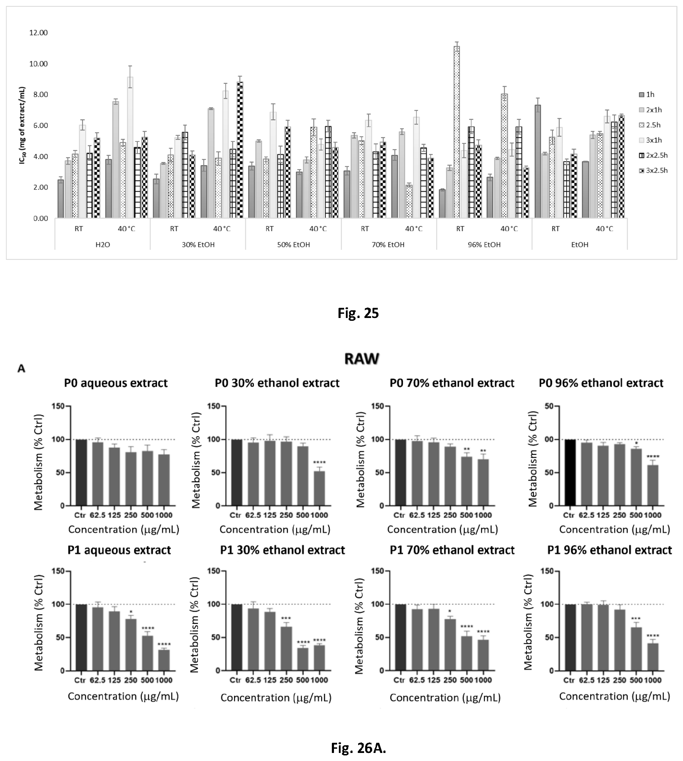

The cork powder extracts were then prepared under the same conditions at 40 °C and room temperature (RT, 20°C) using shorter extraction cycles, namely 3x1h/2.5h in order to establish the best extraction conditions based on the biological potential of the extracts and the sustainability of the extraction process. To predict the effect of temperature, the extraction solvent and the number of extraction cycles, the amount of extract obtained was determined **(Figure 24A).** In addition, the determination of absorbances at 280 and 350 nm served as an indicator of the extraction efficiency of phenolic compounds obtained using polar solvents **(Figures 24B** and **24C).** Finally, the antioxidant activity of each extract was also determined by the DPPH radical scavenging capacity **(****Figure 25****).**

The free radical scavenging activity of 1,1-diphenyl-2-picrylhydrazyl (DPPH) was evaluated according to a previously described method with some modifications. The absorbances were measured at 517 nm in a microplate reader (BioTek Synergy HT Instruments, Winooski, GU, USA). The percentage of inhibition activity was calculated according to the following formula: DPPH radical scavenging effect (%) = [1 - (Abs sample - Abs sample blank /Abs control - Abs blank)]× 100. Ascorbic acid was used as a positive control. The DPPH radical scavenging activity of the extract was expressed as the effective concentration at which the DPPH radical was eliminated by 50% (IC₅₀). The IC₅₀ value was obtained by interpolating the linear regression analysis.

Overall, surprisingly, the extract masses obtained by the extractions at room temperature and at 40 °C were very similar. In both the 1h and 2.5h cycles, there was a greater increase in extract mass from the first to the second extraction than from the second to the third extraction. There was also a decrease in absorbance values, suggesting that the extraction of compounds of interest would decrease with the number of cycles, so it would not be advantageous to carry out multiple extractions.

Overall, all the extracts showed promising antioxidant activity, with IC₅₀ values mostly lower than 10 µg extract/mL **(****Figure 25****).** The comparison of the IC₅₀ values obtained in successive extractions suggested that the antioxidant potential decreased with the number of extractions, as evidenced by the increase in IC₅₀ values in almost all the extraction solvents, regardless of the extraction temperature. Interestingly, for almost all solvents, the antioxidant activity seemed to be better in extracts that were prepared at room temperature and with a one-hour cycle.

Taking into account all these results, as well as the fact that preparing extracts at room temperature is more sustainable and that only one extraction cycle is easier to implement on an industrial scale, it was established that extractions at room temperature with a one-hour cycle were the optimized conditions. When analyzing the DPPH radical scavenging activity of the extracts obtained at room temperature and with a one-hour extraction cycle, the extracts prepared with water, ethanol at 30% (v/v), 70% (v/v) and 96% (v/v) showed promising antioxidant activity and were selected for the following studies.

Following the optimized extraction conditions, new extracts were prepared using not only the P1 cork powder, but also the P0 powder, a darker powder with a larger grain size, obtained at an earlier stage in the production cycle of the cork industry. These extracts were prepared at room temperature for 1 hour with water and with hydroalcoholic solutions of 30% (v/v), 70% (v/v) and 96% (v/v) EtOH.

### Cosmetic/pharmaceutical application of cork extracts

Tests were carried out to assess the potential antioxidant activity using the DPPH method in the extracts prepared from the P0 and P1 powders, according to the optimized conditions, namely 1h of extraction at room temperature using water and the hydroalcoholic mixtures 30% (v/v) ethanol, 70% (v/v) ethanol and 96% (v/v) ethanol as extraction solvents **(Table 18).** Overall, the extracts prepared with 30% (v/v) ethanol proved to be the most active, especially the extract obtained from cork powder P1, which showed the lowest IC₅₀ value. Even so, all the extracts showed promising antioxidant activity, with values of less 10 µg of extract/mL, with the exception of the 96% (v/v) ethanol extract and the aqueous extract of the P0 powder. In addition, the P0 extracts showed lower antioxidant activity compared to the P1 extracts.

**Table 18 - Results of the DPPH radical scavenging activity of the cork powder extracts P0 and P1.**

| | **IC₅₀ (µg extract/mL solution) *** | |
|---|---|---|
| | **PO** | **P1** |
| **H₂O (aqueous extract)** | 15.2± 0.4 | 7.5± 0.1 |
| **30% EtOH** | 7.0± 0.1 | 4.7± 0.2 |
| **70% EtOH** | 7.9± 0.2 | 5.7± 0.1 |
| **96% EtOH** | 11.9± 0.3 | 13.8± 0.3 |

| | | |
|---|---|---|
| * Results expressed as meant standard error, *n=* 3. Ascorbic acid (positive control): ICso= 7.9±0.4 µg/mL. | | |

The composition of the P0 and P1 extracts was then analyzed qualitatively and quantitatively by HPLC. The analytical method was developed according to standards present in cork extracts reported in the literature. The method was validated according to international validation parameters. This technique made it possible not only to understand the effect of the extraction solvent on the profile of the extracts, but also to compare the composition of extracts obtained from different types of cork powder (P0 and P1) and even to try to relate them to the results obtained from the antioxidant activity. The qualitative analysis of the chromatographic bands was based on the UV spectrum and the mass spectrum **(Table 19).** The quantification of the compounds identified in the cork powder extracts P0 and P1 is shown in **Tables 20 and 21.**

Many of the chromatographic bands did not correspond to any of the standards studied, which proves the diversity of the compounds in the extracts. Although the profile of the extracts obtained from the same powder is very similar, there were some differences, particularly in the intensity of some bands depending on the extraction solvent.

**Table 19 - Ultraviolet spectrometry data and mass of the identified peaks**

| **Compound** | **I (nm)** | **ESI/MSⁿ** | |
|---|---|---|---|
| | | **[M-H]⁻** | **MS²** |
| Vescalagina | 280 | 933.06294 | 569.05717/425.01457/300.99906 |
| Gallic acid | | 169.01416 | 125.02438/97.02956/81.03455 |
| Castalagin | | 933.06352 | 569.05746/467.02532/300.99902 |
| Protocatechuic acid | | 153.01921 | 112.98566/110.03281/109.02944 |
| Latifolicin acid C | | 181.05048 | - |
| Protocatechuic aldehyde | | 137.02422 | - |
| Brevifolincarboxylic acid | | 291.01448 | 248.02834/247.02470/202.07883 |
| Aesculetine | | 177.01915 | 149.02441/133.02948/105.03459 |
| Ellagic acid | | 300.99882 | 257.00926/203.08223/202.07886 |
| Coniferous aldehyde | | 177.05563 | - |

In the extracts of coarse cork powder, P0, vescalagin was detected only in the extracts with the highest ethanol content, but at a concentration below the limit of quantification (LQ) of the method validated for the quantification of the extracts. Castalagin, on the other hand, was quantified at higher concentrations in hydroalcoholic extracts, and was the main compound identified in ethanol extracts at 70% EtOH and 96% EtOH. The concentration of ellagic acid followed the same trend, increasing with the amount of ethanol in the extraction solvent, being highest in the 96% ethanol extract. On the other hand, the concentration of gallic acid and latifolicin C was higher in the more polar extracts, i.e. those with a higher water content. The content of the other compounds for all the extraction solvents is shown in **Table 20.**

**Table 20 - Quantification of the compounds identified in the cork powder extracts P0**

| **Compound** | **Concentration (µg/mg dry extract)** | | | |
|---|---|---|---|---|
| | **H₂O** | **30% EtOH** | **70% EtOH** | **96% EtOH** |
| Vescalagina | - | - | <LQ | <LQ |
| Gallic acid | 15.7 | 10.3 | 8.2 | 8.1 |
| Castalagin | 10.4 | 26.5 | 23.6 | 29.1 |
| Protocatechuic acid | 3.8 | 2.3 | 1.7 | 1.5 |
| Latifolicin acid C | 44.9 | 29.1 | 12.8 | 3.2 |
| Protocatechuic aldehyde | 0.5 | 0.5 | 0.5 | 0.6 |
| Brevifolincarboxylic acid | 1.3 | 1.2 | 0.7 | 0.5 |
| Aesculetine | 2.0 | 1.2 | 0.7 | 0.5 |
| Ellagic acid | 7.8 | 12.2 | 19.6 | 27.0 |
| Coniferous aldehyde | <LQ | <LQ | <LQ | <LQ |

| | | | | |
|---|---|---|---|---|
| Lq: Limit of quantification | | | | |

In the fine cork powder extracts, P1, vescalagin was detected in all the extracts, but only quantified in the 30% ethanol extract. In general, castalagin is the majority compound in all the extracts, except in the 96% ethanol extract where ellagic acid is predominant. The concentration of gallic acid was higher in the more polar extracts, i.e. those with a higher water content. The concentration of ellagic acid was higher in the 96% EtOH extract. The concentration of gallic acid and latifolicin C was lower in the P1 extracts compared to the P0 extracts. The concentration of all the compounds identified is shown in **Table 21.**

**Table 21 - Quantification of the compounds identified in the P1 cork powder extracts.**

| **Compound** | **Concentration (µg/mg dry extract)** | | | |
|---|---|---|---|---|
| | **H₂O** | **30% EtOH** | **70% EtOH** | **96% EtOH** |
| Vescalagina | <LOQ | 27.9 | <LQ | <LQ |
| Gallic acid | 7.2 | 6.7 | 5.3 | 4.8 |
| Castalagin | 28.2 | 50.2 | 36.6 | 16.3 |
| Protocatechuic acid | 2.9 | 3.0 | 2.2 | 1.4 |
| Latifolicin acid C | <LQ | 1.7 | 1.0 | - |
| Protocatechuic aldehyde | 0.3 | 0.3 | 0.3 | 0.3 |
| Brevifolincarboxylic acid | 1.3 | 2.0 | 2.1 | 1.5 |
| Aesculetine | 1.4 | 1.2 | 0.7 | 0.6 |
| Ellagic acid | 12.8 | 9.5 | 16.0 | 38.2 |
| Coniferous aldehyde | <LQ | <LQ | <LQ | <LQ |

| | | | | |
|---|---|---|---|---|
| LQ: Limit of quantification | | | | |

The best results for the antioxidant activity of the P1 cork powder extracts can be explained by the greater efficiency in the extraction of phenolic compounds. The extract prepared with 96% ethanol showed the lowest amount of practically all the compounds identified (except ellagic acid), thus contributing to a higher IC₅₀ value.

The P1 extract prepared with 30% ethanol showed the best antioxidant activity, probably due to its higher ellagitannin content. Following the same logic, the P1 extract with 70% ethanol was followed by the highest amount of castalagin, which was in fact the main compound identified in this extract. Next is the P0 extract with 30% ethanol, possibly due to the concentrations of gallic acid and ellagic acid and essentially latifolicin C.

***In vitro* evaluation of the toxicity of the extracts and the effect of the extracts on inflammatory and antioxidant parameters**

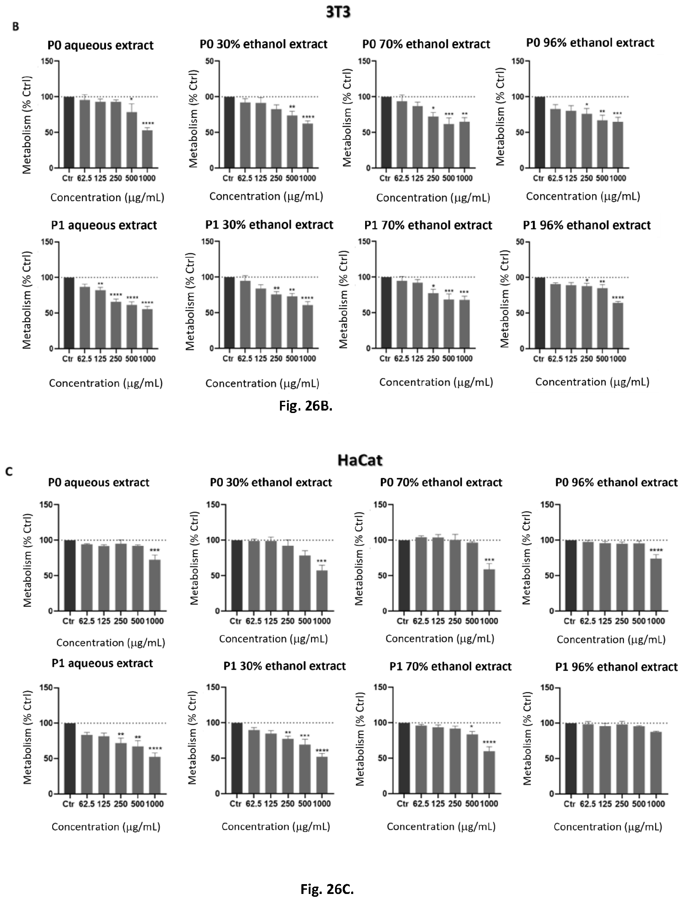

The toxicity of the cork powder extracts prepared from the P0 and P1 powders was assessed according to the optimized conditions, namely 1h of extraction at room temperature using water and the hydroalcoholic mixtures 30% ethanol, 70% ethanol and 96% ethanol on cell lines representative of the skin (human keratinocytes and fibroblasts) and the immune system (macrophages). Cell viability was determined after the cells were exposed to different concentrations (1000, 500, 250, 125 and 62.5 µg/mL) of each of the extracts. Powder extracts P0 and P1 prepared with hydroalcoholic mixtures of 70% ethanol and 96% ethanol were discarded because they were difficult to dissolve in aqueous media. From the results obtained with the remaining extracts, the concentrations of 62.5 and 125 µg/mL were selected as the highest concentrations devoid of toxicity for subsequent tests **(****Figure 26****).**

The potential anti-inflammatory properties of cork powder extracts were subsequently evaluated. To achieve this, macrophages were exposed to cork extracts 30 minutes prior to incubation with the inflammatory stimulus lipopolysaccharide (LPS), an outer membrane component of Gram-negative bacteria, which activates innate immune cells by binding Toll-like receptor 4 (TLR4). In turn, cells such as macrophages and neutrophils synthesize pro-inflammatory mediators.

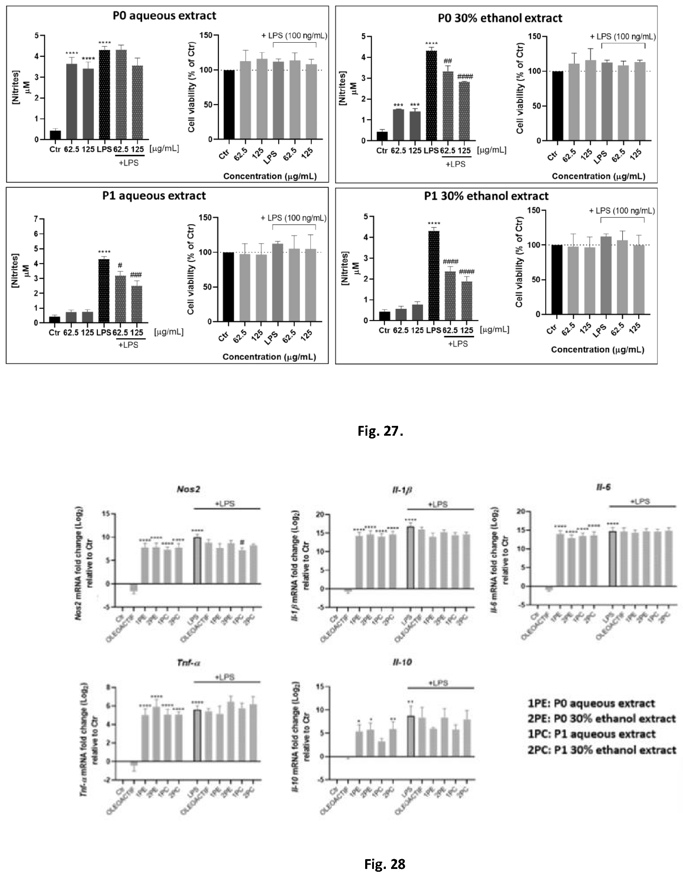

According to **Figure 27****,** the extract derived from P0 obtained with 30% ethanol and the extracts derived from P1, obtained with water or 30% ethanol, inhibited the levels of nitrites induced by LPS in macrophages at the two concentrations tested, with the inhibition being more pronounced at a concentration of 125 µg/ml. As can be seen in **Figure 27****,** the reduction in nitrite levels was not related to lower viability of the cells exposed to LPS. These results show that these extracts have anti-inflammatory properties.

The effect of the extracts on the regulation of genes related to the inflammatory response and oxidative stress in macrophages **(****Figure 28****)** and keratinocytes **(****Figure 29****),** respectively, was then investigated. A commercial cork cosmetic ingredient, DIAM Oleoactif^{®} with the INCI name of "Cocos Nucifera Oil and *Quercus Suber* Bark Extract and Oak Root Extract", which claims anti-inflammatory and antioxidant properties, was also studied to compare results. According to the results, all the extracts increased the expression of the pro-inflammatory genes Nos2 (which encodes for inducible nitric oxide synthase (iNOS)), II1β (which encodes for the protein interleukin-1 beta (IL-1β)), Tnfα (which encodes the tumor necrosis factor-alpha (TNF-α) protein), II6 (which encodes the IL-6 protein) and the anti-inflammatory gene II10 (which encodes the IL-10 protein), in the absence of LPS **(****Figure 28****).** Surprisingly, under an inflammatory environment (i.e. cells exposed to LPS), the aqueous P1 extract induced a significant reduction in the expression of the Nos2 gene (which was not observed with Oleoactif), reinforcing its anti-inflammatory potential.

The expression of the antioxidant genes *HMOX1 (which encodes the protein Heme Oxygenase-1 (HMOX1)), TRX* (which encodes the protein Thioredoxin (TRX)) and *NQ01* (which encodes the protein NAD (P)H dehydrogenase quinone 1 (NQ01)), all proteins with relevant antioxidant activity, was also evaluated **(****Figure 29****).**

While the aqueous P1 and P1-30%ETOH extracts induced a significant increase in the *HMOX1 gene,* only the aqueous P1 extract increased the expression of the *TRX* gene. However, it seems to reduce the expression of *NQ01,* although without statistical significance **(****Figure 29****).** The results obtained suggest an antioxidant role for the aqueous P1 cork extract, an effect not observed for Oleoactif **(****Figure 29****).**

Since the P1 extracts showed relevant anti-inflammatory activity by significantly reducing nitrite levels and *Nos2* gene expression in macrophages exposed to LPS (1PC), and an antioxidant effect by significantly increasing the expression of the *HMOX1* (1PC and 2PC) and *TRX* (1PC) genes in keratinocytes, the P1 extracts were selected for the subsequent experiments.

The effect of cork extracts (and Oleoactif) on iNOS protein levels in macrophages exposed to LPS was then evaluated.

According to **Figure 30****,** both P1 extracts increased iNOS levels *per se,* but only the aqueous extract induced a significant decrease in LPS-induced iNOS protein levels, which is in line with the results obtained for gene expression **(****Figure 28****,** *Nos2* graph).

Since inflammation is closely linked to oxidative stress, the antioxidant potential of cork-derived extracts on oxidative stress triggered by an inflammatory stimulus was also evaluated. Thus, the effect of the extracts on HMOX1 protein levels and on mitochondrial reactive oxygen species (ROS), i.e. superoxide anion (O₂⁻), was determined in macrophages exposed to LPS **(****Figure 31A****),** or to the mitochondrial stress-inducing compound rotenone **(****Figure 31B****),** respectively.

The results obtained showed that the aqueous P1 and P1-30% EtOH extracts increased the antioxidant enzyme HMOX1 in immune cells, in the absence or presence of inflammatory stimuli (macrophages exposed to LPS) **(****Figure 31A****).** These results corroborate the ability of cork extracts to activate the antioxidant pathway in different cellular systems.

### Effect of cork-derived extracts on skin regeneration

The potential of P1 extracts in skin regeneration was also investigated. To achieve this, the expression of the *Col1a1* and *Itgb1* genes (which encode type I collagen and the Integrin beta 1 protein, respectively) **(****Figure 32A****)** and cell migratory capacity (using the *scratch wound assay)* **(****Figure 32B****)** were determined in fibroblasts exposed to cork extracts and the commercial compound Oleoactif. Surprisingly, the extracts significantly decreased the *Col1a1* gene and induced a slight decrease in the expression of the *Itgb1* gene **(****Figure 32A****),** and significantly reduced the migratory capacity of the fibroblasts **(****Figure 32B****).** These results indicate that P1 extracts inhibit cell migration.

### Anti-senescence effect of extracts derived from cork

The anti-senescence capacity of cork extracts was evaluated. Thus, cell senescence was induced in fibroblasts by Etoposide and the cells were subsequently exposed to the aqueous and 30% EtOH extracts of P1 and the commercial compound Oleoactif. As can be seen in **Figure 33****,** both extracts (as well as Oleoactif) reduced the number of senescent cells, suggesting that they have anti-ageing potential.

### Effect of extracts on skin irritation EpiSkin

The skin irritation potential of the aqueous P1 extract was evaluated using a commercial model of reconstructed human epidermis (EpiskinTM), in accordance with the OECD Test 439 *guideline.* According to this skin irritation test, if the average tissue viability is equal to or less than 50%, the sample tested is considered a skin irritant, which was not observed with the formulations containing the aqueous P1 extract **(****Figure 34****).** Thus, this extract shows an adequate safety profile and does not induce skin irritation.

### Cosmetic/pharmaceutical composition comprising cork extract

### Pre-formulation and formulation of anti-aging cosmetic products based on cork extracts

Taking into account the bioactivity, yield of the extract obtained, sustainability of the process and transposition to an industrial scale, the extraction conditions selected were as follows:
- Extraction from P1 cork powder;
- Aqueous solvent extraction;
- 1 extraction cycle of 1 hour;
- Room temperature.

The extracts were obtained from 100 ml of solvent for 2.5 g of powder, according to the following procedure:
- Stir in Erlenmeyer for 1 hour;
- Filtration with 1.2 µm filter paper;
- Rotavapor evaporation;
- Freeze-drying.

Any freeze-drying process can be applied to the product of the present disclosure. For example, in one embodiment the following protocol was applied: after evaporation in rotavapor, the resulting residue is frozen at -80 °C, vacuum is applied (0.01-0.1 mbar) and then the water is removed from the frozen residue by sublimation.

At this stage, the extract was produced in quantity in order to carry out its physicochemical characterization. Around 6 g of extract were obtained.

Characterization tests were carried out on the aqueous extract of cork powder P1. This characterization included the determination of organoleptic characteristics (color, odor and appearance), pH determination, chemical profile, determination of heavy metals, evaluation of microbiological quality and evaluation of the bioactivity of the extract obtained.

### Determination of organoleptic characteristics

The color of the extract was characterized using a colorimeter (ChromaMeter CR-400), recording the brightness values and chromaticity coordinates **(Table** 22). As this is an ingredient of natural origin and it is not possible to control the color of each batch of extract obtained, this property is extremely important for assessing the variability between batches and quality control, as the color obtained will directly influence the color of the formulation into which it will be incorporated.

**Table 22: L*a*b color parameters of EAP1 (n=3).**

| Aqueous extract | |
|---|---|
| Color | L*= 50.39±0.15 |
| | a*= 5.71±0.26 |
| | b*= 15.55±0.47 |

To determine the pH of the extract, a 10% (w/v) aqueous dispersion was prepared. The pH was measured in the filtrate using an HI 2211 pH/ORP/°C meter (Hanna Instruments, Woonsocket, RI, USA). The measurements were taken in triplicate **(Table 23).**

**Table 23: pH of the aqueous extract n=3.**

| Aqueous extract | |
|---|---|
| pH | 5.30±0.006\| 18 °C |

### Determination of heavy metal content

According to Regulation 1223/2009 on cosmetic products, the presence of heavy metals in ingredients can only be detected in trace quantities. Although the regulation does not mention legal limits, it is mandatory to analyze the content present.

The content of heavy metals was found to be compatible with use in cosmetic products, and their presence was not the result of intentional addition during the process of obtaining the extract **(Table 24).**

**Table 24: Heavy metal content (mean ± SD) of the aqueous extract (ppm) n=3.**

| Aqueous extract | |
|---|---|
| Heavy Metals (ppm) | |
| As | 0.36±0.057 |
| Cd | 0.13±0.003 |
| Hg | 0.02±0.009 |
| Pb | 1.63±0.030 |

### Obtaining the chemical profile - Infrared Spectroscopy

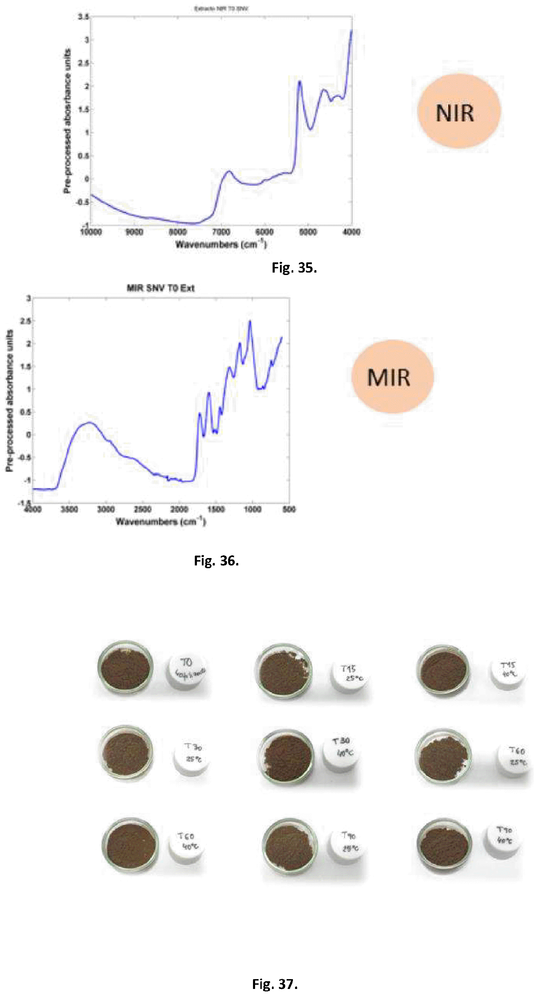

Near-infrared (NIR) and mid-infrared (MIR) spectra were obtained for the aqueous extract **(****Figures 35 and 36****).** Obtaining the spectra at time zero allowed to obtain a physicochemical profile of the obtained extract. Although NIR and MIR alone are not sufficient to assess the composition of the extract, they are of particular interest for evaluating potential changes over time and quality control.

### Pre-formulation studies

### Microbiological quality assessment

The microbiological quality analysis protocol was prepared in accordance with ISO 21148:2017 and the European Pharmacopoeia 10. The sample was prepared by diluting 0.1 g of aqueous extract in 0.9 mL of saline solution. Serial dilutions up to 10⁻³ were also prepared. Aliquots of 100 µL of the original samples and dilutions were spread, in duplicate, on the surface of Tryptic Soy Agar (TSA) medium for the isolation of bacteria, and incubated for 24-48 hours at 30-35 ± 2 °C.

The microbiological quality of the aqueous extract was examined in accordance with ISO 21148:2017 and the European Pharmacopoeia 10, specifically the chapter "Microbiological examination of non-sterile products". The total microbiological count, for the conditions used in the test, was lower than the legal limits (2.00x10³CFU/g) established by ISO.

### Evaluation of bioactivity and total phenolic compounds

As shown, the aqueous cork extract obtained from the P1 by-product powder has antioxidant activity.

In order to characterize the new extract obtained, the antioxidant activity was evaluated using the DPPH method and the total phenolic content (TPC) was determined according to the Folin-Ciocalteu method, with some modifications **(Table 25).** TPC was calculated using a calibration curve drawn with gallic acid and expressed in mg of gallic acid equivalent per g of dry extract (mg GAE/g of extract).

**Table 25: Total phenolic content and DPPH scavenging activity (IC₅₀) of the aqueous extract at time zero (T0, start of the stability study). n=3**

| **Test** | **T0** |
|---|---|
| DPPH (IC50=µg extract/mL) | 5.79±1.07 |
| Total Phenolic Compounds (mg of gallic acid/g of extract) | 421.11±30.0 |

### Solubility test

The solubility of the aqueous extract was assessed according to OPPTS guidelines 830.7840, in order to evaluate potential solvents to be included in cosmetic product base formulations. Three solvents were selected: water, glycerin and 1,3 - butylene glycol. The extract proved to be soluble in water and glycerin at a ratio of 1g/L and insoluble in butylene glycol.

### Stability study of aqueous cork extract

The stability study took place over 90 days and with storage at two temperatures: 25 and 40 °C. The tests were carried out as shown in **Table 26.**

**Table 26: Summary of the aqueous extract stability protocol.**

| | **T0 (0 days)** | **T15 (15 days)** | | **T30 (30 days)** | | **T60 (60 days)** | | **T90 (90 days)** | |
|---|---|---|---|---|---|---|---|---|---|
| | 25^{°}C | 25^{°}C | 40^{°}C | 25 ^{°}C | 40 ^{°}C | 25^{°}C | 40 ^{°}C | 25 ^{°}C | 40 ^{°}C |
| Appearance | x | x | x | x | x | x | x | x | x |
| Color | x | x | x | x | x | x | x | x | x |
| Odor | x | x | x | x | x | x | x | x | x |
| pH | x | x | x | x | x | x | x | x | x |
| MIR | x | x | x | x | x | x | x | x | x |
| NIR | x | x | x | x | x | x | x | x | x |
| Microbiological quality | x | | | | | | | x | x |
| DPPH | x | x | x | x | x | x | x | x | x |

| | | | | | | | | | |
|---|---|---|---|---|---|---|---|---|---|
| X: test carried out | | | | | | | | | |

The storage container used was a glass flask, protected with aluminum foil on the outside in order to minimize the potential effect of light.

Through the photographic records taken during the stability study, it can be concluded that, macroscopically, there were no organoleptic changes, particularly in terms of color **(****Figure 37****).**

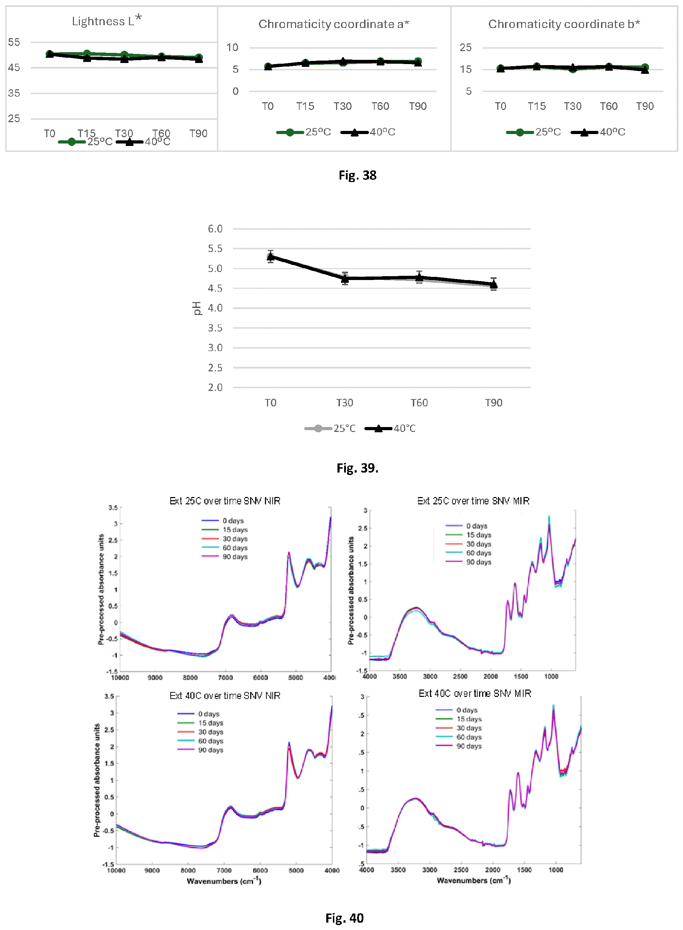

At the same time, the color of the aqueous extract was analyzed more objectively using a colorimeter, which allow to obtain information relative to the parameters of luminosity (L*) and chromaticity coordinates a* and b* **(****Figure 38****).** It was therefore confirmed that no considerable differences were detected over the analysis time, regardless of the temperature. The aqueous extract had a woody odor, which remained unchanged throughout the analysis period.

The pH remained relatively stable, between pH 4.5 and 5.3 throughout the analysis period and regardless of the temperature **(****Figure 39****).**

Near-infrared (NIR) and mid-infrared (MIR) spectra were also obtained for the aqueous extract **(****Figure 40****)** initially and throughout the stability test (T0, T15, T30, T60 and T90) for both temperatures (25 and 40 °C) and showed that the chemical composition of the extract remained unchanged over time, since the spectra obtained did not show the appearance of new bands or the disappearance of existing bands. The overlapping of the spectra also reveals their stability **(****Figure 40****).**

Microbiological quality was assessed at time 0 of the test and at 90 days for samples stored at 25 °C and 40 °C. The test was carried out inside a horizontal laminar flow chamber in order to reduce possible risks of sample contamination. The sample of aqueous P1 extract (1:10 dilution) was prepared and duly homogenized. Serial dilutions (10⁻¹, 10⁻² and 10⁻³) were then prepared in duplicate. The total aerobic microorganism count was 5.00 x 10² CFU/g, 48 hours after the start of the analysis.

The procedure was repeated at day 90 of the stability study, for both the sample stored at 25 °C and 40 °C. In these cases, the count of aerobic mesophilic microorganisms was 3.00 x 10² CFU/g and 5.00 x 10² CFU/g, respectively. In all cases, this was lower than the limits established in ISO 21148:2017 (2 x10³CFU/g).

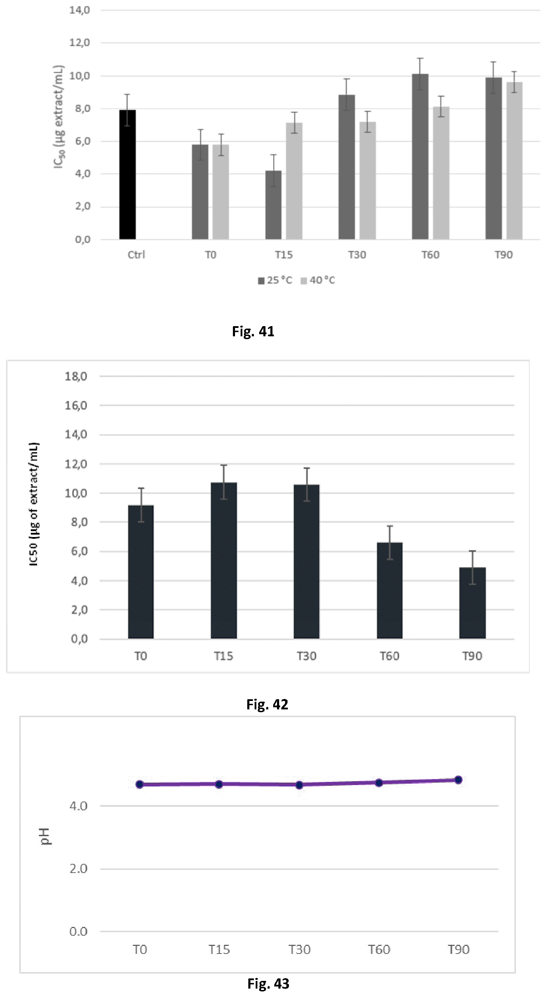

The antioxidant activity of the aqueous extract was also assessed during the stability test using the DPPH assay. The IC₅₀ values were evaluated at 0, 15, 30, 60 and 90 days for the P1 aqueous extract stored at 25 and 40 °C. Ascorbic acid was used as a positive control (IC₅₀ = 7.9 ± 0.4 µg/mL) **(****Figure 41****).**

The aqueous extract proved to be moderately stable at temperatures of 25 and 40 °C with regard to the DPPH scavenging effect.

The IC₅₀ of the extract at 25 °C ranged from 5.79 ± 1.07 µg/mL at time 0, to 9.92 ± 1.20µg/mL at time 90 days. For the extract stored at 40 °C, the IC₅₀ ranged between 5.79 ± 1.07 µg/mL at time 0 to 9.64 ± 1.20µg/mL at time 90 days.

The antioxidant activity of the extract remained present at the end of the 90 days of storage at both temperatures.

Total phenolic compounds were also assessed at time 90 **(Table 27).**

**Table 27: Total phenolic content and DPPH scavenging activity (IC₅₀) of the aqueous extract. Comparison between t0 and t90 (n=3).**

| *Aqueous extract* | | |
|---|---|---|
| **Test** | T0 | T90 (25 °C) |
| **DPPH (IC50=µg of extract/mL)** | 5.79±1.07 | 9.92±1.20 |
| **Total Phenolic Compounds (mg of gallic acid/g of extract)** | 421.11±30.0 | 253.2±19.97 |

The stability of the extract was also assessed in a 3.5 wt % glycerol solution. The IC₅₀ values at 25 °C ranged from 9.17 ± 0.34 µg/mL (T0) to 4.91 ± 1.30 µg/mL (T90). The results showed that the incorporation of the extract into this solution is quite stable, with only a slight variation in DPPH scavenging capacity over the 90 days **(****Figure 42****).**

The pH values remained constant, at values close to pH 5, throughout the test **(****Figure 43****).**

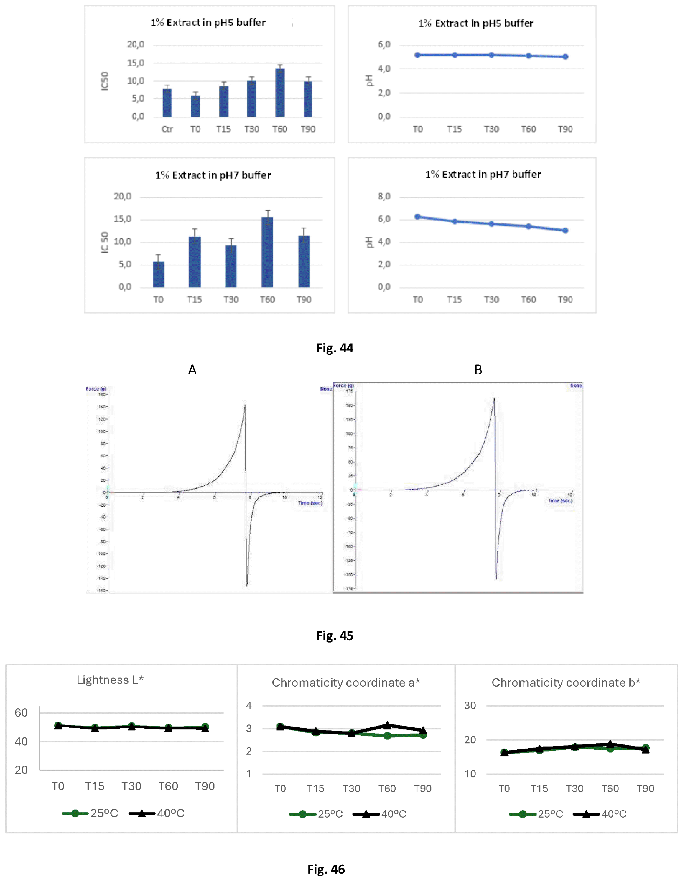

In order to assess the stability of the aqueous extract at different pH values, two buffer solutions were prepared at pH 5 and 7. The extract proved to be more stable in the pH 5 buffer solution **(****Figure 44****).**

### Base formulation for extract incorporation test (EAP1)

The base formulation was selected in order to study the stability of the developed ingredient when incorporated into formulations of cosmetic/pharmaceutical products, with a face cream being selected for incorporation of the aqueous extract (EAP1).

In order to verify the physicochemical stability over time, the respective protocol for evaluating the stability of the formulation was also developed.

### Stability of Formulation with Aqueous Cork Extract

With regard to the formulations developed, the physical stability protocol was implemented, namely with the evaluation of the rheological behavior.

From the formulations tested, face cream formulation CR002 was selected to incorporate 1 wt. % lyophilized extract, as it was more pleasant to the touch and had a better visual appearance **(Table 28).**

**Table 28: Composition of formulation CR002 and respective preparation procedure.**

| Phase | Ingredients | Amount (wt%) |
|---|---|---|
| **A** | Olivatis^{®} 18 _{(polyglyceryl-6 esters of olive oil, sodium stearoyl lactate, cetostearyl alcohol)} | 5.00 |
| **A** | Miglyol 812 _{(caprylic/capric triglyceride)} | 15.00 |
| **A** | Lanette O _{(cetostearyl alcohol )} | 2.00 |
| **B** | Xanthan gum | 0.15 |
| **B** | Propylene glycol | 2.00 |
| **B** | Water | qs 100 |
| **C** | Cork extract | 1.00 |
| **C** | Euxyl^{®} _{(phenoxyethanol+ Benzyl alcohol+ Ethylhexylglycerin+ Tocopherol)} | 1.00 |
| Procedure: | | |
| 1. Heat phases A and B to 65-70 °C. | | |
| 2. Add the phases to the ultraturrax and leave to cool - under stirring for 15 minutes. | | |
| 3. Switch to paddle stirring (slower) until 40 °C is reached. | | |
| 4. Add phase C and mix for 10 minutes. | | |
| (Note: remove part of the water to dissolve the extract and subtract it from the remaining water to be added) | | |

The following tests were carried out over the 3 months of the trial **(Table 29).**

**Table 29: Summary of the stability protocol for the formulation with extract incorporated at 1 wt%**

| | T0 | T15 | | T30 | | T60 | | T90 | |
|---|---|---|---|---|---|---|---|---|---|
| | 25^{°}C | 40^{°} C | 25^{°}C | 40 °C | 25^{°}C | 40 °C | 25^{°}C | 40^{°} C | 25^{°}C |
| Appearance | x | x | x | x | x | x | X | x | x |
| Color | x | x | x | x | x | x | X | x | X |
| Odor | x | x | x | x | x | x | X | x | x |
| pH | x | x | x | x | x | x | X | x | x |
| Spreadability | x | | | | | | | | |
| Microbiologic al quality | x | | | | | | | x | x |
| Viscosity | x | | | | | | | x | x |
| Thixotropy | x | | | | | | | x | x |

| | | | | | | | | | |
|---|---|---|---|---|---|---|---|---|---|
| **X: Test carried out** | | | | | | | | | |

### Initial characterization of the formulation

After selecting the base formulation (face cream reference CR002) lyophilized cork extract was incorporated into it at a concentration of 1 wt%.

Before starting the stability test, the base formulation and the formulation with extract were subjected to a mechanical stress test in order to preliminarily assess their stability (centrifugation at 3500 rpm for 30 minutes). As there was no phase separation, we consider the formulation to be stable.

In order to characterize the formulation with incorporated extract, its spreadability was evaluated using a texturometer.

The ease with which the conical probe moves downwards in the cream translates into the product's spreading capacity. As soon as the probe comes into contact with the sample, the force increases steadily until a maximum value is reached. The probe then reverses direction and withdraws from the sample. In this case, the force measured is negative because the formulation adheres to the probe, resisting its movement out of the sample container **(****Figure 45****).** It can be concluded that storage temperature does not contribute to marked differences in the spreadability of the aqueous cork extract formulation analyzed.

### Evaluation of the formulation's organoleptic characteristics during the stability test

The color of the formulations was assessed using a colorimeter **(Tables 30 and 31).** Due to the extract's characteristic brown color, it gave a light brown hue to the base formulation, which was white.

**Table 30: Color parameters of the base formulation.**

| **Color** | | | |
|---|---|---|---|
| **Sample (t0)** | L* | A* | B* |
| **Base CR002 (1)** | 67.55 | -0.50 | 1.50 |
| **Base CR002 (2)** | 67.55 | -0.51 | 1.50 |
| **Base CR002 (3)** | 67.55 | -0.50 | 1.49 |
| **Mean±SD** | 67.55±0.00 | -0.50±0.01 | 1.50±0.01 |

**Table 31: Color parameters of the base formulation with aqueous extract.**

| Color | | | |
|---|---|---|---|
| **Sample (t90)** | L* | A* | B* |
| **CR002 Extract 1 wt% (1)** | 51.35 | 3.09 | 16.31 |
| **CR002 Extract 1wt % (2)** | 51.35 | 3.09 | 16.32 |
| **CR002 Extract 1 wt% (3)** | 51.34 | 3.09 | 16.31 |
| **Mean±SD** | 51.35±0.00 | 3.09±0.00 | 16.31±0.01 |

The color of the formulations stored at 25 and 40 °C was analyzed over 90 days in order to understand whether this parameter remained stable **(****Figure 46****).** In fact, it was concluded that both the luminosity and the chromaticity coordinates A and B of the formulation with the extract remained practically unchanged throughout the analysis period, regardless of the temperature.

### pH assessment

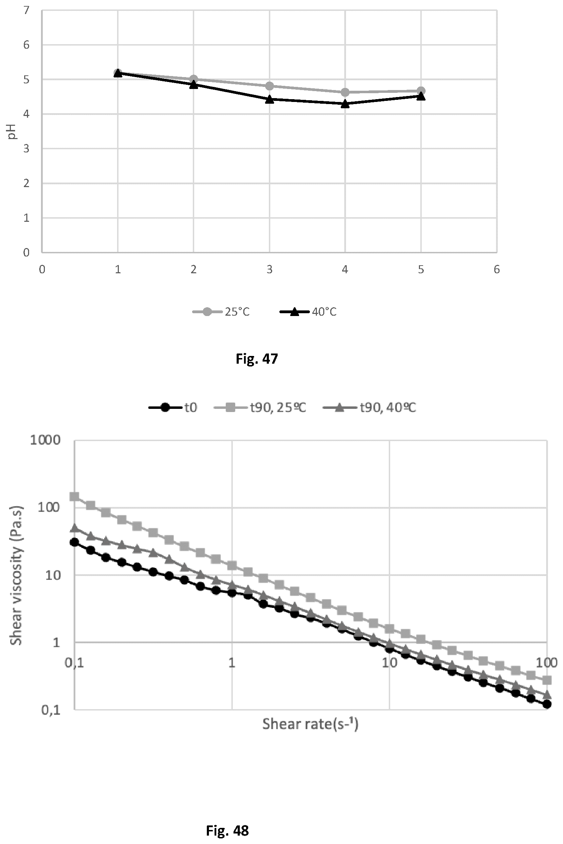

Evaluation of the pH over the 3 months of the stability test **(****Figure 47****)** showed that the pH decreased slightly over time (minimum pH value 4.3). However, it remains at values considered suitable for use on the skin.

### Microbiological quality assessment

The microbiological quality of the formulation under study was evaluated initially and after 90 days of storage at 25 °C and 40 °C.

The microbiological evaluation of cosmetic products is mandatory for batch release and must be carried out in accordance with ISO standards 21148:2017 and 17516:2014, and Pharmacopoeia 10.

The manufacture of cosmetic products must follow Good Manufacturing Practices and these must mitigate contamination, and the microbiological quality of a cosmetic product should not depend exclusively on its preservative system. Therefore, the assessment of microbiological quality requires the use of a neutralizing system, which inactivates the preservative allowing microorganisms to grow if they are present in the formulation.

Samples of the formulations stored at 25 °C and 40 °C were prepared under the following conditions:
- Sample in sterile serum (1:10)
- Sample in neutralized serum (1:10)
- Sample in sterile serum enriched in TSB (1:10)
- Sample in neutralized serum enriched in TSB (1:10)

As controls, sterile serum, neutralized serum and TSB were also incubated to ensure that they were not a source of contamination for the sample.

The neutralizing system was selected according to the manufacturer's instructions for the preservative system used. After inoculating and incubating the plates, respectively undiluted and diluted at 10⁻¹, 10⁻² and 10⁻³, the microbiological count was found to be within the legal limits set by ISO (2x10³UFC/g).

### Rheological behavior

### Flow

The flow was evaluated initially and after 90 days **(****Figure 48****).**

Apparent viscosity is a variable that characterizes rheologically a system . Evaluating this parameter helps to determine whether a product has the appropriate consistency or fluidity and can indicate whether stability is adequate, i.e. it provides an indication of the product's behavior over time.

The behavior exhibited by CR002 cream over time (non-Newtonian fluid) is confirmed when the power law model is applied. The value of the flow index (n) indicates that the viscosity decreases as the shear rate increases **(Table 32).**

**Table 32: Parameters of the Power Law model.**

| | t0 (25°C) | t90 (25°C) | t90 (40°C) |
|---|---|---|---|
| *K* | 4.728 | 12.624 | 7.628 |
| *n* | -0.780 | -1.047 | -0.803 |
| *R²* | 0.9932 | 0.999 | 0.9960 |

The samples remained stable and without phase separation, although there were slight changes in the rheological behavior.

The aqueous P1 cork extract and the formulation in which it was incorporated remained stable during the 3-month stability test. The extract proved to maintain its antioxidant activity throughout the stability test, thus allowing us to conclude on the viability of this ingredient as an active ingredient in cosmetic products.
The term "comprises" or "comprising" whenever used herein is intended to indicate the presence of stated characteristics, elements, integers, steps and components, but not to preclude the presence or addition of one or more other characteristics, elements, integers, steps and components, or groups thereof. The following claims define additional embodiments of the present description.

## Claims

1. Cork powder for use in cosmetics or medicine comprising cork particles with a particle size of less than 100 µm, and wherein the color has values of L* between 45-55; of a* between 6-10 and of b* between 12-16, as measured by the CIELAB system.

2. Cork powder according to the previous claim comprising cork particles with a particle size of less than 63 µm; preferably ranging between 16-63 µm; more preferably 20-40 µm.

3. Cork powder according to any one of the previous claims wherein the cork powder is subjected to 180 °C for at least 30 minutes; preferably 30 minutes.

4. Cork powder according to any one of the previous claims wherein the oil absorption capacity of the cork powder is at least 4 times higher than that of kaolin.

5. Method of obtaining the cork powder according to any one of claims 1-4 comprising the following steps:
supplying cork powder comprising cork particles with a particle size of less than 100 µm; preferably less than 63 µm;
adding water and stirring for at least 15 minutes in order to obtain a dispersion of cork powder;
filtering the cork powder dispersion through a porous glass filter in order to obtain clean cork powder;
drying the clean cork powder at least at 50 °C for at least 1 hour to obtain dry cork powder;
heating the dry cork powder at 180 °C for at least 30 minutes.

6. Cork powder according to any one of claims 1-4 or method according to claim 5 wherein the cork powder is selected from: crushing powder, cleaning powder, powder from granulometric separations, powder from the finishing of agglomerate panels, powder from the finishing of cork stoppers and discs; powder from the finishing of natural cork stoppers, or mixture thereof; preferably powder from granulometric separations.

7. Aqueous extract of cork powder for cosmetic or medical use comprising:
castalagin, at a concentration of more than 10 µg/mg (dry extract);
gallic acid, at a concentration of more than 2 µg/mg (dry extract);
ellagic acid, at a concentration of more than 4 µg/mg (dry extract).

8. Extract according to the previous claim wherein the extract is obtained from the cork powder according to any one of the previous claims 1-4.

9. Extract according to any one of claims 7-8 comprising:
castalagin, at a concentration ranging between 10-60 µg/mg (dry extract), preferably between 10-50 µg/mg (dry extract);
gallic acid, at a concentration ranging between 2-20 µg/mg (dry extract), preferably between 2-15 µg/mg (dry extract);
ellagic acid, at a concentration ranging between 4-30 µg/mg (dry extract), preferably between 4-20 µg/mg (dry extract).

10. Cosmetic or pharmaceutical composition comprising cork powder according to any one of claims 1-4 or the aqueous extract of cork powder according to any one of the previous claims 7-9.

11. Composition according to the previous claim comprising 1-7 wt % cork powder; preferably 4-6 wt% cork powder; more preferably 5 wt%.

12. Composition according to the previous claim comprising 0.1-4 wt% cork powder; preferably 1-3 wt% cork powder; more preferably 2 wt%.

13. Composition according to any one of the previous claims 11-12 further comprising titanium dioxide; preferably 1-15 wt % titanium dioxide; more preferably 8-12 wt%.

14. Composition according to the previous claim comprising:
1-7 wt % cork powder; preferably 5 wt %;
1-15 wt% titanium dioxide; preferably 10 wt%.

15. Composition according to any one of the previous claims 11-14 further comprising:
a mixture of glyceryl monostearate, cetostearyl alcohol, cetyl palmitate and coco-glycerides; preferably 2-8 wt%;
ethoxylated cetostearyl alcohol (ceteareth-12); preferably 0.1-3 wt%;
medium-chain triglycerides; preferably 4-12 wt%;
cetearyl isononanoate; preferably 4-12 wt%.

16. Composition according to any one of the previous claims 11-15 wherein the sun protection factor is at least 40; preferably between 40-80.

17. Composition according to any one of the previous claims 11-16 further comprising:
2-8 wt% of a mixture of polyglyceryl-6 esters of olive oil, sodium stearoyl lactate, cetostearyl alcohol;
4-12 wt%of dicaprylyl ether;
1-4 wt% of squalene;
1-3 wt% of caprylic/capric acid triglycerides;
1-4 wt% of stearic acid;
1-3 wt% of sweet almond oil;
0.1-0.5 wt% of xanthan gum;
1-3 wt% of Propanediol;
2-8 wt% of Glycerin;
0.1-2 wt% of a mixture of phenoxyethanol, benzyl alcohol, ethylhexylglycerin and tocopherol.

18. Composition according to the previous claim wherein the color of the cosmetic composition has an L* between 40-54; an a* between 4-8 and a b* between 10-17, measured by the CIELAB system.

19. Composition according to the previous claim 10 comprising 0.1-2 wt% aqueous extract of cork powder; preferably 0.1-1 wt%; more preferably 0.5 -1 wt%.

20. Composition according to the previous claim further comprising:
4-6 wt% of a mixture of polyglyceryl-6 esters of olive oil, sodium stearoyl lactate, cetostearyl alcohol;
10-20 wt% of triglycerides of capric acid and/or caprylic acid;
1-3 wt% of cetostearyl alcohol;
0.1-0.2 wt% of xanthan gum;
1-3 wt% of propylene glycol;
0.1-2 wt% of a mixture of phenoxyethanol, benzyl alcohol, ethylhexylglycerin and tocopherol.

21. Composition according to any one of the previous claims 19-20 wherein the color of the composition has an L* between 48-55; an a* between 2 and 7, a b* between 15-20, measured by the CIELAB system.

22. Composition according to any one of the previous claims 10-21 wherein the composition is selected from a list comprising: solution, serum, cream, lotion, gel, hydrogel, oleogel, oil, soap, shampoo, dry shampoo, spray, ointment, paste, mousse or body wash; preferably cream.

23. Use of the cork powder according to any one of claims 1-4 or of the composition according to any one of the previous compositions 11-18 as an oil-absorbing agent.

24. Use of the cork powder according to any one of claims 1-4 or of the composition according to any one of the previous compositions 11-18 as a pigment; preferably as a cosmetic pigment.

25. Use of the cork powder according to any one of claims 1-4 or of the composition according to any one of the previous compositions 11-18 as a sun protection factor enhancing agent, preferably wherein the enhancing factor is at least 2 times; more preferably the enhancing factor ranges between 2-3 times.

26. Use of the cork powder according to any one of claims 1-4 or of the composition according to any one of the previous compositions 11-18 as an ultraviolet radiation blocking agent.

27. Cork powder according to any one of claims 1-4 or composition according to any one of the previous compositions 12-21 for use in medicine or veterinary.

28. Cork powder according to any one of claims 1-4 or composition according to any one of the previous compositions 11-18 for use in the prevention and treatment of skin diseases; preferably skin diseases caused by exposure to the sun.

29. Use of the extract according to any one of claims 7-9 or of the composition according to any one of claims 19-22 as a skin ageing preventive or retarding agent.

30. Use of the extract according to any one of claims 7-9 or of the composition according to any one of claims 19-22 as an anti-senescent agent.

31. Use of the extract according to any one of claims 7-9 or of the composition according to any one of claims 19-22 as an antioxidant agent.

32. Extract according to any one of claims 7-9 or composition according to any one of claims 19-22 for use in medicine; preferably for use in the prevention or treatment of inflammation.

33. Extract according to any one of claims 7-9 or composition according to any one of claims 19-22 for use as an anti-inflammatory; preferably topical anti-inflammatory.

34. Method of obtaining the extract as described in any one of the previous claims 7-9 comprising the following steps:
supplying cork powder comprising cork particles with a particle size of less than 0.5 mm;
adding an aqueous solvent to the cork powder in a mass ratio of cork powder: aqueous solvent that ranges between 1:20 and 1:80, and stirring for at least 30 minutes to obtain a mixture comprising the aqueous extract of cork powder;
filtering the mixture comprising the aqueous extract of cork powder to obtain an aqueous extract of cork powder.

35. Method according to the previous claim comprising an additional step of drying and freeze-drying the aqueous extract of cork powder in order to obtain a freeze-dried aqueous extract of cork powder.

36. Method according to any one of the previous claims 34-35 wherein the granulometry of the cork particles is less than 250 µm; preferably between 63-200 µm; more preferably 100-200 µm.

37. Method according to any one of the previous claims 34-36 wherein the mass ratio of cork powder: aqueous solvent ranges between 1:30 and 1:50; preferably, the mass ratio of cork powder: aqueous solvent is 1:40.

38. Method according to any one of the previous claims 34-37 wherein the stirring step of the aqueous solvent and the cork powder ranges between 500-1000 rpm; preferably 700 rpm.

39. Method according to any one of the previous claims 34-38 wherein the aqueous solvent is water.

40. Method according to any one of claims 34-39 wherein the method is carried out at room temperature.

41. Method according to any one of the previous claims 34-40 wherein the stirring time of the aqueous solvent and the cork powder ranges from 30 - 120 minutes; preferably 60 minutes.

42. Method according to any one of the previous claims 34-41 wherein the filtration step is carried out with a glass fiber filter membrane; preferably with a diameter of 47 mm and a pore size of 1.2 µm.

43. Method according to any one of the previous claims 34-42 wherein the extraction step is carried out in a single extraction cycle.

44. Extract according to any one of the previous claims 7-9 or method according to any one of claims 34-43 wherein the cork powder used is selected from: crushing powder, cleaning powder, powder from granulometric separations, powder from the finishing of agglomerate panels, powder from the finishing of cork stoppers and discs; powder from the finishing of natural cork stoppers, or mixture thereof; preferably powder from granulometric separations.
